(19) Europäisches Patentamt
European Patent Office
Office européen des brevets

(11) EP 3 097 904 B1

(12) **EUROPEAN PATENT SPECIFICATION**

(45) Date of publication and mention of the grant of the patent:
**14.08.2019 Bulletin 2019/33**

(21) Application number: **15305836.7**

(22) Date of filing: **29.05.2015**

(51) Int Cl.:
*A61K 8/33* (2006.01) *A61K 8/34* (2006.01)
*A61K 8/35* (2006.01) *A61K 8/36* (2006.01)
*A61K 8/368* (2006.01) *A61K 8/41* (2006.01)
*A61K 8/49* (2006.01) *A61Q 19/02* (2006.01)
*A61K 8/97* (2017.01) *C07D 311/16* (2006.01)
*C07C 217/18* (2006.01) *C07D 295/088* (2006.01)

(54) **DEPIGMENTING AGENTS TO LIGHTEN THE SKIN**

DEPIGMENTIERUNGSMITTEL ZUR AUFHELLUNG DER HAUT

AGENTS DE DÉPIGMENTATION POUR ÉCLAIRCIR LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.11.2016 Bulletin 2016/48**

(73) Proprietor: **Affichem**
**31400 Toulouse (FR)**

(72) Inventors:
- **DE MEDINA, Philippe**
  **31170, Colomiers (FR)**
- **BIZE, Cécile**
  **31100, Toulouse (FR)**
- **RIVES, Arnaud**
  **31400, Toulouse (FR)**
- **PAILLASSE, Michaël**
  **31100 Toulouse (FR)**
- **GENOVESE, Salvatore**
  **86039, Termoli (CB) (IT)**
- **EPIFANO, Francesco**
  **65013, Città Sant'Angelo (PE) (IT)**

(74) Representative: **Cabinet Plasseraud**
**66, rue de la Chaussée d'Antin**
**75440 Paris Cedex 09 (FR)**

(56) References cited:
**WO-A1-2012/079154 WO-A1-2012/094638**
**DE-A1- 2 160 136**

- **DATABASE CA [Online] CHEMICAL ABSTRACTS SERVICE, COLUMBUS, OHIO, US; 1962, Bates, R.B et al.: "Stereochemistry of terpenoid double bonds of the type CH2CMe:CHCH2X", XP002749463, Database accession no. 57:42983 & CHEMISTRY & INDUSTRY, 1962, pages 1020-1021,**
- **JI, Q. ET AL.: "Benzothieno[3,2-b]indole derivatives as potent selective estrogen receptor modulators", BIIORGANIC & MEDICINAL CHEMISTRY LETTERS, vol. 15, 2005, pages 2891-2893, XP002749464,**
- **BISHT, S.S. ET AL.: "Synthesis and optimization of antitubercular activities in a series of 4-(aryloxy) phenyl cyclopropyl methanols", EUROPEAN JOURNAL OF MEDICINAL CHEMISTRY, vol. 45, 2010, pages 5965-5978, XP002749465,**
- **YADAV, Y. ET AL.: "Design, synthesis and bioevaluation of novel candidatte selective estrogen receptor modulators", EUROPEAN JOURNAL OF MEDICINAL CHEMISTRY, vol. 46, 2011, pages 3858-3866, XP002749466,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 097 904 B1

## Description

The present invention relates to a method for lightening a skin or a mucous membrane with a depigmenting agent, a cosmetic use of said depigmenting agent, to cosmetic compositions comprising said agent, and to new depigmenting agents.

The whiteness of the skin is particularly important for Asian populations which associate this criterion to beauty and social rank. Hyperpigmented spots such as ephelis, lentigines, "cafe au lait" spots and nævi are universally considered unaesthetic. The relative demand for depigmenting agents is thus considerable. In human beings, the color of skin is determined by diverse pigments such as blood hemoglobin, carotenoids, bilirubin and melanin. The latter is by far the main factor that determines skin coloring. The predominant physiological role of melanin is to protect the skin against ultraviolet rays which enhance the ageing process and the risk of developing skin cancer. Solar exposition triggers melanogenesis which is due to an adaptive response of melanocytes which are cells located in the basal layer of the epidermis that specialize in melanin biogenesis. When they are irradiated by UV rays, keratinocytes secrete $\alpha$-MSH (melanocyte-stimulating hormone) which is bound to melanocytes via receptors and thus induces transduction pathways and activates transcription factors that stimulate melanin production. Melanin is stored in vesicular structures called melanosomes which are subsequently matured in the melanocytes and transported in the multiple melanocyte indentations as described in Nature Reviews, Molecular Cell Biology, Vol. 2, October 2001, p. 738-748. The transported melanosomes are then transferred to the skin keratinocytes which lead to melanin migrating through the successive layers of skin to the strateum corneum and the consecutive darkening of the skin. Hyperpigmentation results from an excess of melanin in the skin due to extrinsic factors such as photosensitizing agents, physical and/or chemical lesions, and inflammation; or intrinsic factors such as endocrinal, metabolic and/or genetic disorders. Melanogenesis is a complex process that implies successive enzymatic transformations of tyrosine catalyzed by tyrosinase, TRP1 (Tyrosinase Related Protein 1) and TRP2 (Tyrosinase Related Protein 2). Tyrosinase has been identified as the key enzyme in melanin production. The transcription of genes coding melanogenic enzymes is regulated by Mitf transcription factor which is itself controlled by different signaling pathways such as Wnt, P38, MAPK or AMPc.

As such, the main strategies used to lighten the skin are based on inhibiting melanogenesis enzymes or the regulators that express them, blocking the transfer of melanosomes in keratinocytes or stimulating desquamation. Currently marketed depigmenting agents mainly target tyrosinase inhibition and include kojic acid, arbutin, hydroquinone, vitamin C and azelaic acid. However, these compounds exhibit one of the following drawbacks: low stability, low efficiency, undesirable secondary effects such as skin irritation, toxicity towards melanocytes or even carcinogen activity. These negative aspects have substantially limited the use of these compounds as depigmenting agents. Toxicity issues have even led Japanese authorities to prohibit kojic acid and hydroquinone has been banned by the European Committee ($24^{th}$ Dir 2000/6/EC) from cosmetic compositions to lighten the skin. The use of vitamin C and arbutin has been limited by their instability with respect to respectively oxidation and certain enzymes present in the skin.

WO 2012/094638 discloses a method for the modification of melanin distribution using a substituted benzaldehyde. US 3,712,947 discloses sun-screening compositions comprising coumarin ethers.

As such, there is a need for new depigmenting agents that are stable, efficient and non-toxic.

An object of the present invention is therefore a method for lightening a skin or a mucous membrane in a patient, said method comprising topically administering to said patient an effective amount of a depigmenting agent of general formula (I):

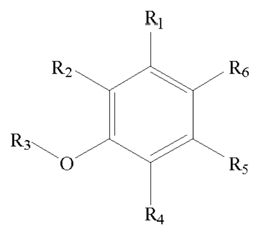

(I)

wherein

- $R_1$ is H or hydroxy;
- $R_2$ is H or methoxy;
- $R_3$ is $C_5$-$C_{15}$ alkenyl optionally substituted by hydroxy or $R_3$ is $-CH_2-CH_2-NR_7R_8$;
- $R_4$ is H or methoxy;
- $R_5$ is H or hydroxy;

$R_6$ is selected in the group consisting of $-CH_2-Ph$; -COOH; -CH=CH-COOH; -CH=CH-CHO; $-CH=CH-CH_2OH$; -CO-CH=CH-Ph; wherein Ph is a phenyl optionally substituted by hydroxy; $-CH_2CH_2COOH$; $-(CH_2)_n-OH$ with n=1, 2 or 3; or $R_5$ and $R_6$ form, with the carbon atoms to which they are attached, an unsubstituted fused 5,6-dihydropyran-2-one ring or an optionally substituted fused 2,3-dihydro-1,4-naphthalenedione system;
$R_7$ and $R_8$ are each independently $C_1$-$C_3$ alkyl or $R_7$ and $R_8$ form, with the nitrogen atom to which they are attached, a five-membered ring.

Another object of the present invention is the cosmetic use of a depigmenting agent of general formula (I) as defined above, to induce lightening of a skin or a mucous membrane, to improve the uniformity and/or clarity of a skin or a mucous membrane, and/or to prevent, reduce and/or remove hyperpigmented spots.

Yet another object of the present invention is a cosmetic composition comprising, in a cosmetically acceptable carrier, a depigmenting agent of general formula (I) as defined above, a plant extract containing said depigmenting agent of general formula (I), and mixtures thereof, wherein said composition further comprises at least one UV-filter,
with the proviso that said at least one UV-filter does not correspond to general formula (II)

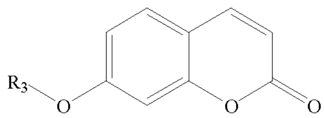

(II)

wherein $R_3$ is $C_5$-$C_{15}$ alkenyl optionally substituted by hydroxy.

## Description of figures

Figure 1 shows the effect of umbelliprenin and kojic acid (KA) on the level of basal melanin in a cell line of murine melanocytes.

Figure 2 shows the effect of PBPE and tesmilifene (Tes) on the level of basal melanin in a cell line of murine melanocytes.

Figure 3 shows the effect of umbelliprenin (Umb), PBPE and tesmilifene (Tes) on melanin synthesis in presence of $\alpha$-MSH in a cell line of murine melanocytes.

Figure 4 shows the effect of umbelliprenin (Umb), PBPE and tesmilifene (Tes) on melanin synthesis in a cell line of murine melanocytes that have been previously irradiated with UV rays.

Figure 5 shows the effect of umbelliprenin (Umb), PBPE and tesmilifene (Tes) alone or in combination on melanin synthesis in a cell line of normal murine melanocytes.

Figure 6 shows the melanin level and the tyrosinase enzymatic activity measured in lysates of murine melanocytes obtained from murine melanocytes that have been previously treated with umbelliprenin (Umb), PBPE, kojic acid or tesmilifene (Tes).

Figure 7 is a photography of a cell pellet of murine melanocytes treated with a solvent carrier or by umbelliprenin.

Figure 8 shows the effect of umbelliprenin, PBPE and tesmilifene on the expression de genes coding for tyrosinase, Mitf, TRP1 and TRP2.

Figure 9 shows the effect of umbelliprenin, PBPE and tesmilifene on the viability of murine melanocytes.

## Definitions

The term "topically administering to said patient" means that the depigmenting agent is applied to the area which needs to be treated on a patient, i.e. to the skin or to a mucous membrane that needs to be lightened.

The term "an effective amount" means the amount of a depigmenting agent that, when administered to the patient for lightening the skin or a mucous membrane, is sufficient to effect such lightening. The effective amount will vary depending on the depigmenting agent, the severity of the pigmentation and the age, weight, etc... of the patient.

The term "$C_1$-$C_3$ alkyl" means a branched or unbranched saturated hydrocarbon radical of formula $C_nH_{2n+1}$ wherein n is an integer equal to 1, 2 or 3.

The term "$C_5$-$C_{15}$ alkenyl" means a branched or unbranched unsaturated hydrocarbon radical comprising 5, 6, 7, 8, 9, 10, 11, 12, 13, 14 or 15 carbon atoms and 1, 2 or 3 carbon-carbon double bonds.

The term "$C_5$-$C_{15}$ alkenyl optionally substituted by hydroxy" means a $C_5$-$C_{15}$ alkenyl as defined above wherein

a hydrogen atom is optionally replaced with a hydroxy group.

The term "unsubstituted $C_5$-$C_{15}$ alkenyl" means a $C_5$-$C_{15}$ alkenyl as defined above which comprises only hydrogen and carbon atoms.

The term "fused 5,6-dihydropyran-2-one ring" means a ring having the following formula:

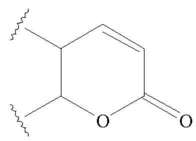

wherein the undulated lines represent the points of attachment of the 5,6-dihydropyran-2-one ring to the ring of general formula (I).

The term "fused 2,3-dihydro-1,4-naphthalenedione system" means a system having the following formula:

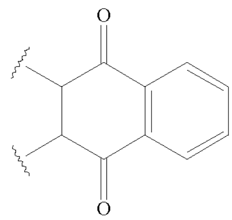

wherein the undulated lines represent the points of attachment of the fused 2,3-dihydro-1,4-naphthalenedione system to the ring of general formula (I) (optional substituents on the 2,3-dihydro-1,4-naphthalenedione system are not shown).

The term "five-membered ring" means a cyclic compound comprising a ring of 5 atoms.

The term "cosmetic composition" means a composition intended to be administered topically, said composition exhibiting a color, a smell and a texture that do not generate inacceptable discomfort to the user, such as itching, tightness and redness of the skin.

The term "cosmetically acceptable carrier" means a carrier that is not toxic and may be applied on the skin or mucous membrane of a mammal.

**Method**

The method for lightening a skin or a mucous membrane in a patient according to the present invention comprises topically administering to said patient an effective amount of a depigmenting agent.

The depigmenting agent used in method of the present invention corresponds to general formula (I) below:

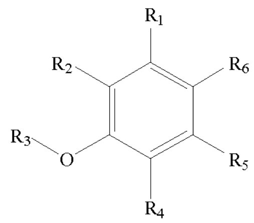

(I)

wherein

$R_1$ is H or hydroxy;
$R_2$ is H or methoxy; $R_3$ is $C_5$-$C_{15}$ alkenyl optionally substituted by hydroxy or $R_3$ is $-CH_2-CH_2-NR_7R_8$;
$R_4$ is H or methoxy;
$R_5$ is H or hydroxy;
$R_6$ is selected in the group consisting of $-CH_2-Ph$; -COOH; -CH=CH-COOH; -CH=CH-CHO; $-CH=CH-CH_2OH$; -CO-CH=CH-Ph; wherein Ph is a phenyl optionally substituted by hydroxy; $-CH_2CH_2COOH$; $-(CH_2)_n-OH$ with n=1, 2 or 3; or $R_5$ and $R_6$ form, with the carbon atoms to which they are attached, an unsubstituted fused 5,6-dihydropyran-2-one ring or an optionally substituted fused 2,3-dihydro-1,4-naphthalenedione system;

$R_7$ and $R_8$ are each independently $C_1$-$C_3$ alkyl or $R_7$ and $R_8$ form, with the nitrogen atom to which they are attached, a five-membered ring.

According to a first embodiment of the method of the present invention, the depigmenting agent corresponds to general formula (I) wherein
$R_1$ is H;
$R_2$ and $R_4$ are each independently H or methoxy;
$R_3$ is unsubstituted $C_5$-$C_{15}$ alkenyl;
$R_5$ is H or hydroxy; and
$R_6$ is selected in the group consisting of $-CH_2-Ph$; -COOH; -CH=CH-COOH; -CH=CH-CHO; $-CH=CH-CH_2OH$; -CO-CH=CH-Ph; wherein Ph is a phenyl optionally substituted by hydroxy; $-CH_2CH_2COOH$; $-(CH_2)_n-OH$ with n=1, 2 or 3.

In particular, the $R_3$ group of the depigmenting agent of the first embodiment of the method of the present invention is selected in the group consisting of unsubstituted branched alkenyl with 5 carbon atoms and 1 unsaturation, unsubstituted branched alkenyl with 10 carbon atoms and 2 unsaturations and unsubstituted branched alkenyl with 15 carbon atoms and 3 unsaturations. More particularly, said $R_3$ group may be $(-CH_2-CH=C(CH_3)-CH_2)_m-H$ wherein m is 1, 2 or 3. Even more particularly, said $R_3$ group may be selected from isopentenyl (m=1), geranyl (m=2) or farnesyl (m=3).

According to a second embodiment of the method of the present invention, the depigmenting agent corresponds to general formula (I) wherein
$R_1$, $R_2$, $R_4$ and $R_5$ are H;
$R_3$ is $-CH_2-CH_2-NR_7R_8$;
$R_6$ is $-CH_2-Ph$; and
$R_7$ and $R_8$ are each independently $C_1$-$C_3$ alkyl or $R_7$ and $R_8$ form, with the nitrogen atom to which they are attached, a five-membered ring.

In particular, the $R_7$ and $R_8$ groups of the depigmenting agent of the second embodiment of the method of the present invention may each be ethyl or $R_7$ and $R_8$ may form, with the nitrogen atom to which they are attached, a pyrrolidine ring.

According to a third embodiment of the method of the present invention, the depigmenting agent corresponds to general formula (I) wherein
$R_1$, $R_2$ and $R_4$ are H;
$R_3$ is $C_5$-$C_{15}$ alkenyl optionally substituted by hydroxy;
$R_5$ and $R_6$ form, with the carbon atoms to which they are attached, an unsubstituted fused 5,6-dihydropyran-2-one ring.

As such, the depigmenting agent of the third embodiment of the method of the present invention may correspond to general formula (II)

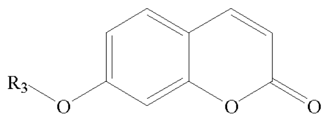

(II)

wherein $R_3$ is $C_5$-$C_{15}$ alkenyl optionally substituted by hydroxy.

In particular, the $R_3$ group of the depigmenting agent of the third embodiment of the method of the present invention is selected in the group consisting of branched alkenyl with 5 carbon atoms, 1 unsaturation and substituted by one hydroxy group, unsubstituted branched alkenyl with 12 to 15 carbon atoms and two or three unsaturations. More particularly, said $R_3$ group may be an unsubstituted branched alkenyl with 15 carbon atoms and 3 unsaturations. Even more particularly, said $R_3$ group may be selected from hydroxyisopentenyl ($-CH_2-CH=C(CH_3)-CH_2-OH$) or farnesyl ($[-CH_2-CH=C(CH_3)-CH_2]_3-H$).

According to a fourth embodiment of the method of the present invention, the depigmenting agent corresponds to general formula (I) wherein
$R_1$ is OH;
$R_2$ and $R_4$ are H;
$R_3$ is unsubstituted $C_5$-$C_{15}$ alkenyl;
$R_5$ and $R_6$ form, with the carbon atoms to which they are attached, an optionally substituted fused 2,3-dihydro-1,4-naphthalenedione system.

Preferably, said fused 2,3-dihydro-1,4-naphthalenedione system is substituted with at least one substituent selected from OH and Me, more preferably, said 2,3-dihydro-1,4-naphthalenedione system is substituted by at least one OH group and at least one Me group.

As such, the depigmenting agent of the fourth embodiment of the method of the present invention may correspond to general formula (III)

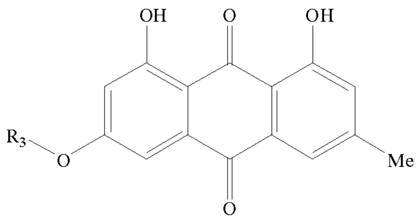

(III)

wherein $R_3$ is unsubstituted $C_5$-$C_{15}$ alkenyl.

In particular, the $R_3$ group of the depigmenting agent of the fourth embodiment of the present invention is selected in the group consisting of unsubstituted branched alkenyl with 5 carbon atoms and 1 unsaturation, unsubstituted branched alkenyl with 10 carbon atoms and 2 unsaturations and unsubstituted branched alkenyl with 15 carbon atoms and 3 unsaturations. More particularly, said $R_3$ group may be $(-CH_2-CH=C(CH_3)-CH_2)_p-H$ wherein p is 1, 2 or 3. Even more particularly, said $R_3$ group may be selected from isopentenyl (p=1), geranyl (p=2) or farnesyl (p=3).

The depigmenting agent used in the method of the present invention may notably be selected in the group consisting of:

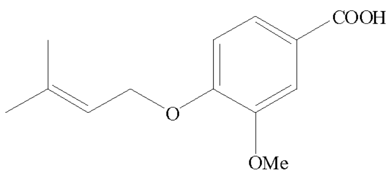
,
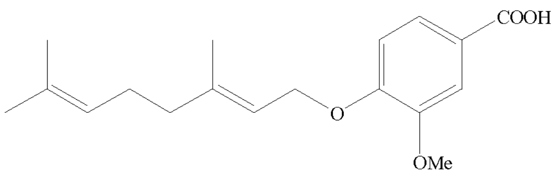
,

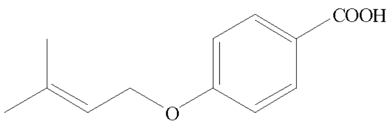
,
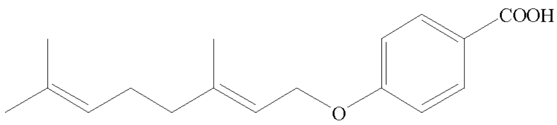
,

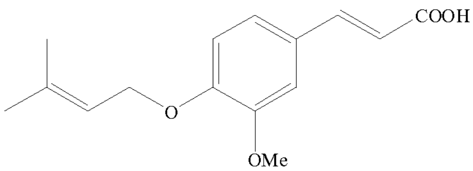
,
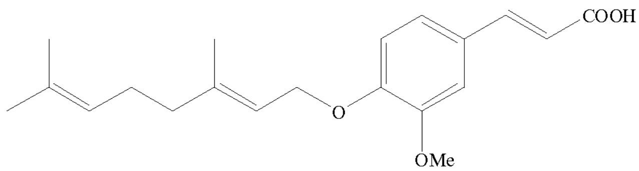
,

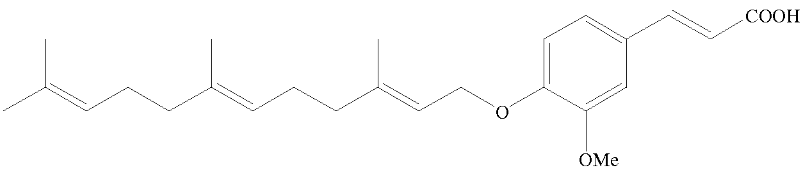
,

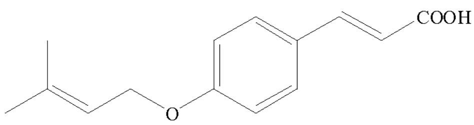
,
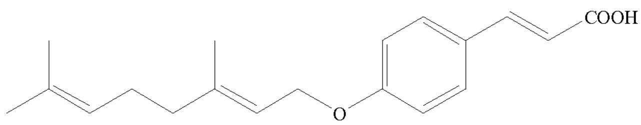
,

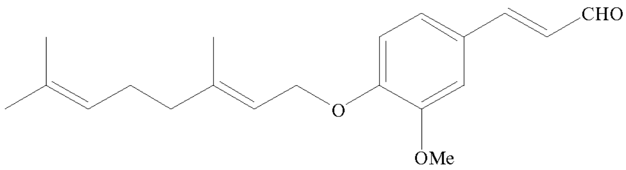,

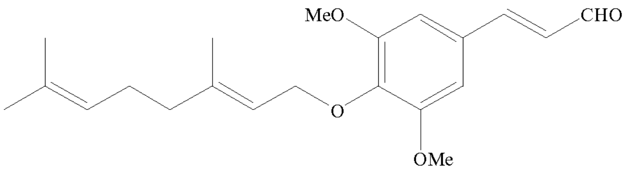, 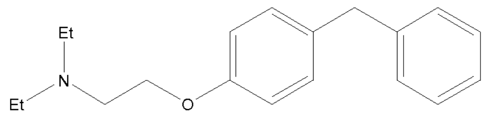,

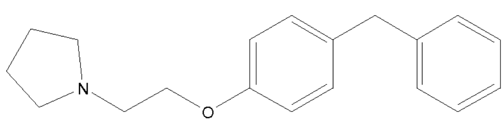, 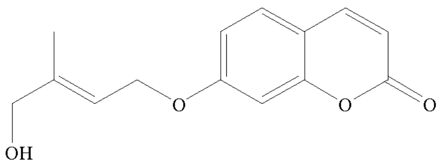,

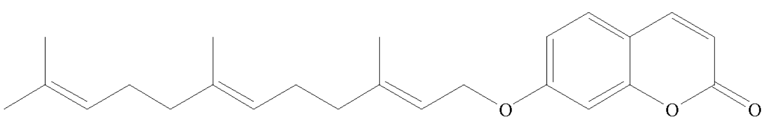,

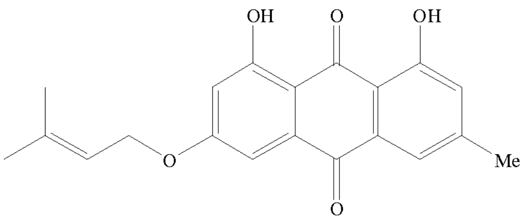,

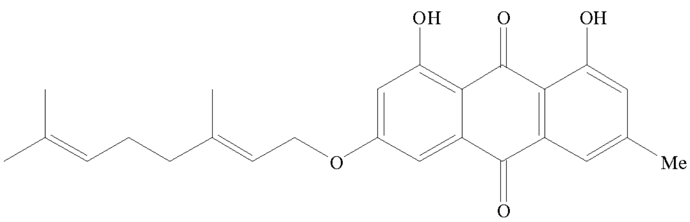,

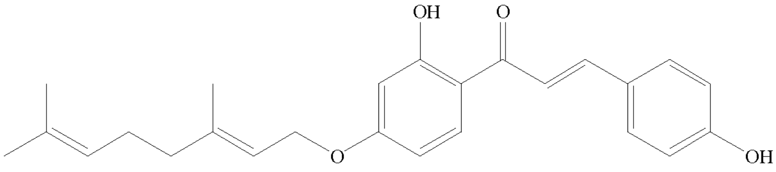,

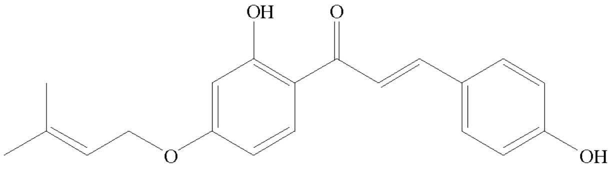,

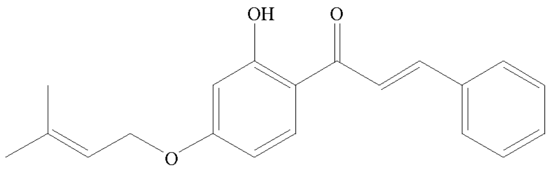
,
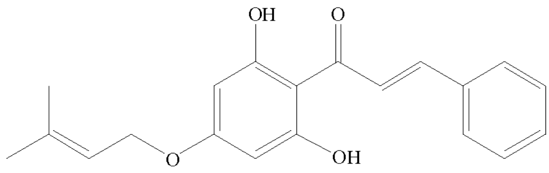
,

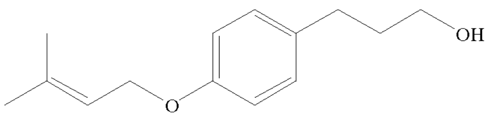
,
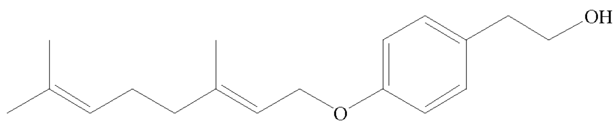
,

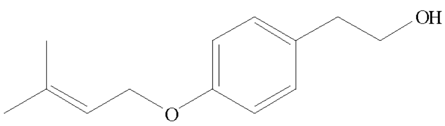
,
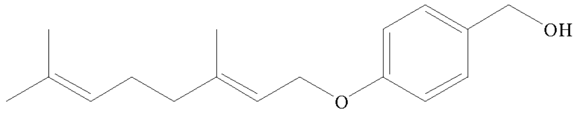
,

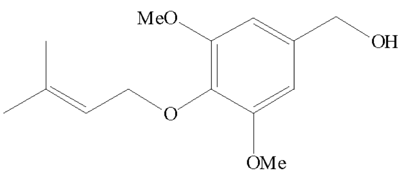
,
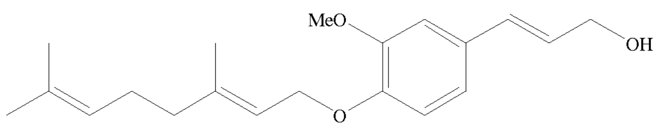
,

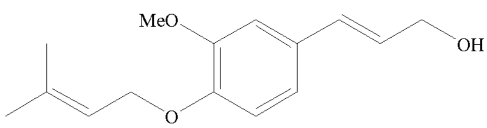
,
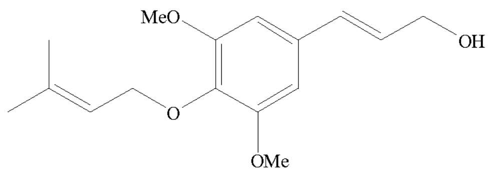
,

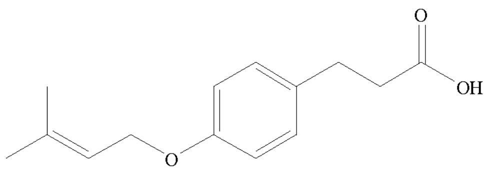
,

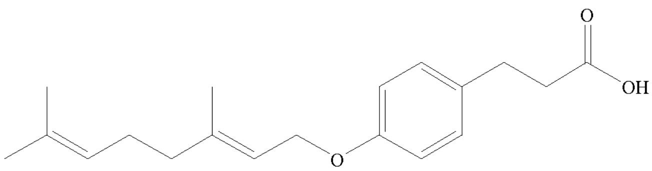
,
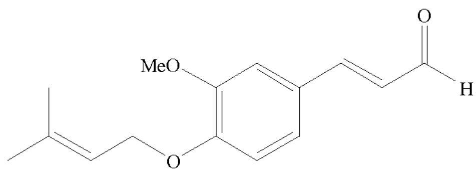
,

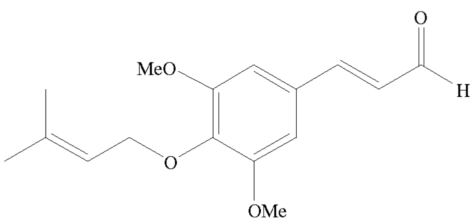
,
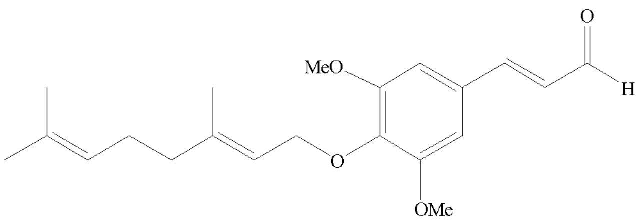
,

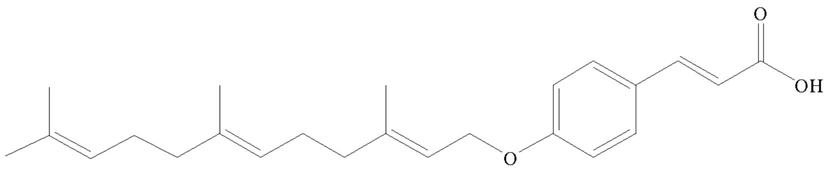
,

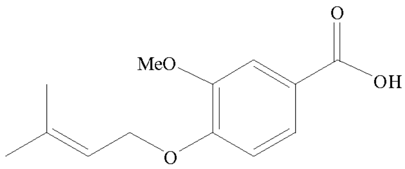
,
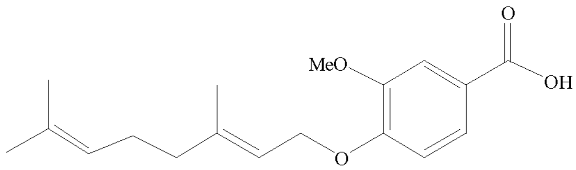
,

and mixtures thereof.

The depigmenting agent of general formula (I) used in the method of the present invention may be prepared according to various methods well known in the art.

For example, a depigmenting agent of general formula (I) in which $R_3$ is a $C_5$-$C_{15}$ alkenyl can be obtained according to the synthetic route detailed hereinafter:

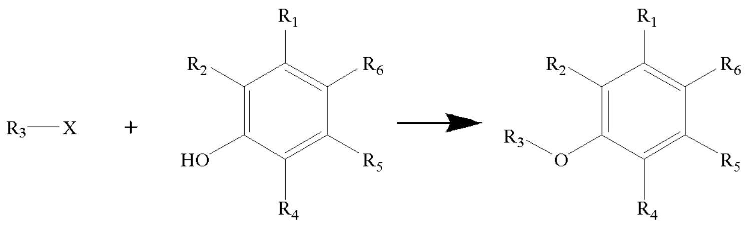

(I)

wherein $R_1$, $R_2$, $R_4$, $R_5$ and $R_6$ are as previously defined and X is Cl or Br.

The method of the present invention comprises topically administering to the skin or a mucous membrane of a patient a depigmenting agent of general formula (I) as defined above. In particular, the depigmenting agent may be administered on at least an area of the skin, for example the face, the neck, the arms, the hands, the legs, the feet, the chest. The depigmenting agent may also be applied on at least an area of a mucous membrane, for example the lips, the genital area, the anus. The depigmenting agent may also be applied on an area of the skin that is hyperpigmented, i.e. on a hyperpigmented spot, for example a chloasma, freckles, an ephelis, a nævi, lentigines, in particular lentigo simplex, age-related spots and remaining islands of skin colour in vitiligo.

According to an embodiment of the method of the present invention, the topical administration of the depigmenting agent is carried out at least once a day, in particular at least once a day during at least a week, more particularly at least once a day during at least a month.

## Cosmetic use

The present invention also relates to the cosmetic use of a depigmenting agent to induce lightening of a skin or a mucous membrane, to improve the uniformity and/or clarity of a skin or a mucous membrane, and/or to prevent, reduce and/or remove hyperpigmented spots.

The depigmenting agent of the cosmetic use according to the present invention corresponds to general formula (I) as previously defined for the method of the present invention.

According to an embodiment of the cosmetic use of the present invention, said depigmenting agents of general formula (I) may be used for preventing, reducing and/or removing hyperpigmented spots selected in the group consisting of chloasma, freckles, ephelis, nævi, lentigines, in particular lentigo simplex, age-related spots, and remaining islands of skin colour in vitiligo, and mixtures thereof.

## Cosmetic composition

The cosmetic composition of the present invention comprises, in a cosmetically acceptable carrier, a depigmenting agent, a plant extract containing said depigmenting agent, and mixtures thereof, and at least one UV-filter with

the proviso that said at least one UV-filter does not correspond to general formula (II) as defined below.

The depigmenting agent of the cosmetic composition according to the present invention corresponds to general formula (I) as previously defined for the method of the present invention.

The cosmetic composition of the present invention comprises a depigmenting agent of general formula (I), a plant extract containing said depigmenting agent of general formula (I), and mixtures thereof. In particular, the cosmetic composition may comprise a mixture of at least two depigmenting agent of general formula (I) according to the invention or a mixture of at least two plant extracts containing said depigmenting agent of general formula (I).

In particular, the plant extract containing a depigmenting agent of general formula (I) that may be introduced in the cosmetic composition of the present invention may be selected in the group consisting of a plant extract from the genus *Ferula,* in particular *Ferula szowitsiana, Ferula sinkiangensis, Ferula assa-foetida, Ferula tunetana, Ferula flabelliloba, Ferula fukanensis, Ferula persica* and *Ferula aitchisonii;* a plant extract from the genus *Angelica,* in particular *Angelica sylvestris, Angelica komarovii, Angelica pubescens, Angelica pachycarpa, Angelica decursiva, Angelica archangelica* and *Angelica ursina;* a *Pimpinella anisum* extract; an *Anethum graveolens* extract;, *a Ferulago campestris extract,* a *Cicuta virosa* extract, a *Seseli annuum* extract; a *Peucedanum zenkeri* seed extract; a *Thapsia villosa* fruit extract; a *Scandix pecten-veneris* extract; a *Scabiosa comosa* extract; a *Heracleum sphondylium* extract; an *Ammi majus* extract; a *Xanthogalum sachokianum* extract; a *Thamnosa montana* extract; a *Ligusticum seguieri* extract; and mixtures thereof.

Plant extracts comprising a depigmenting agent of general formula (I) can, for example, be obtained by solid-liquid extraction of at least one part of a plant selected in the group consisting of a plant from the genus *Ferula,* in particular *Ferula szowitsiana, Ferula sinkiangensis, Ferula assa-foetida, Ferula tunetana, Ferula flabelliloba, Ferula fukanensis, Ferula persica* and *Ferula aitchisonii;* a plant from the genus *Angelica,* in particular *Angelica sylvestris, Angelica komarovii, Angelica pubescens, Angelica pachycarpa, Angelica decursiva, Angelica archangelica* and *Angelica ursina; Pimpinella anisum; Anethum graveolens; Ferulago campestris; Cicuta virosa; Seseli annuum; Peucedanum zenkeri* seeds; *Thapsia villosa* fruits; *Scandix pecten-veneris; Scabiosa comosa*; *Heracleum sphondylium; Ammi majus; Xanthogalum sachokianum; Thamnosa montana; Ligusticum seguieri;* and mixtures thereof; with a solvent. In particular, said solvent may be selected from water, alcohol, alkane, ethyl acetate, dimethylformamide, dimethylsulfoxide, and mixtures thereof.

According to an embodiment of the present invention, the cosmetic composition may comprise 0.0001% to 50%, in particular 0.1% to 20%, more particularly 1% to 10%, by weight of the depigmenting agent of general formula (I) based on the weight of the composition.

The cosmetic composition of the present invention also comprises a cosmetically acceptable carrier.

The cosmetically acceptable carrier may notably be selected in the group consisting of a solvent, an oil, a fatty substance, and mixtures thereof.

The solvent used as a cosmetically acceptable carrier may be selected in the group consisting of water, monoalcohols, glycols, and mixtures thereof.

Examples of suitable alcohols that may be used as a cosmetically acceptable carrier include ethanol, isopropanol, diacetone alcohol, 2-butoxyethanol, cyclohexanol, n-propanol, n-butanol; ethers of propylene glycol that are liquid at room temperature such as monomethyl ether of propylene glycol, monomethyl ether acetate of propylene glycol, mono-n-butyl ether of dipropylene glycol; and mixtures thereof. Examples of suitable glycols that may be used as a cosmetically acceptable carrier include propylene glycol, polyethylene glycol, and mixtures thereof.

Examples of suitable oils that may be used as a cosmetically acceptable carrier include vegetable oils such as linseed oil, rapeseed oil, sunflower oil, soybean oil, olive oil, palm oil, castor oil, corn oil, grapeseed oil, jojoba oil, sesame oil, almond oil, cottonseed oil, alfafa oil, coconut oil, safflower oil, peanut oil and apricot oil; animal oils, such as perhydrosqualene and fish liver oils; mineral oils, such as hydrogenated polyisobutene, liquid paraffin and mixtures of hydrocarbons; synthetic oils obtained by reacting an alcohol or a polyol with one or more fatty acids, such as purcelline oil, isopropyl myristate and ethylhexyl palmitate; fluorinated oils, such as perfluoropolyethers; silicone oils such as cyclomethicones and dimethicones; and mixtures thereof.

Examples of suitable fatty substances that may be used as a cosmetically acceptable carrier include unsaturated fatty acids; vegetable butter such as shea butter and cocoa butter; waxes such as paraffin wax, carnauba wax, beeswax ozokerite, polyethylene wax; silicone waxes; and mixtures thereof.

The cosmetic composition may comprise 5% to 99.9999%, in particular 20% to 99%, more particularly 40% to 95%, by weight of the cosmetically acceptable carrier based on the weight of the composition.

The cosmetic composition of the present invention comprises at least one UV-filter. A UV-filter is a compound that is either able to reflect and scatter UV rays or to absorb UV rays and to dissipate their energy. UV-filters are useful to prevent sun-induced repigmentation of the skin. Furthermore, UV-filters may help to prevent the photodegradation of the depigmenting agent of general formula (I).

The at least one UV-filter that is introduced in the composition of the present invention does not correspond to general formula (II):

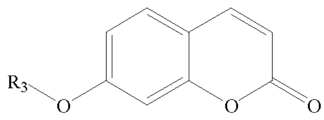

(II)

wherein $R_3$ is $C_5$-$C_{15}$ alkenyl optionally substituted by hydroxy.

In an embodiment of the present invention, the at least one UV-filter is selected in the group consisting of an organic UV-filter, a mineral UV-filter, and mixtures thereof.

Examples of suitable organic UV-filters that may be introduced in the composition of the present invention include derivatives of dibenzoylmethane, such as butyl methoxydibenzoylmethane; esters of cinnamic acid such as ethylhexyl methoxycinnamate; salicylates; β-β'-diphenylacrylates; triazines; phenylbenzotriazoles; and mixtures thereof.

Examples of suitable mineral UV-filters that may be introduced in the composition of the present invention include mineral oxides, such as titanium dioxide and zinc oxide, that may be in the form of pigments or nanopigments, that may be coated or uncoated.

The cosmetic composition may comprise 0.1% to 40%, in particular 1% to 10%, more particularly 2% to 5%, by weight of the at least one UV-filter based on the weight of the composition.

The cosmetic composition of the present invention may further comprise a second depigmenting agent, or mixtures thereof. Indeed, the second depigmenting agent may have additive or synergistic effect with the depigmenting agent of general formula (I) to reduce skin melanin or to prevent the accumulation of skin melanin. Said second depigmenting agent may notably be selected in the group consisting of inhibitors of melanogenesis enzymes, copper chelating agents, antioxidants, inhibitors of melanosome transfer from melanocytes to keratinocytes, exfoliants, inhibitors of alpha-MSH production, inhibitors of endothelin production, inhibitors of adrenocorticotropic hormone (ATCH) production, and mixtures thereof.

Examples of suitable second depigmenting agents that may be introduced in the composition of the present invention are selected in the group consisting of hydroquinone, arbutin, azelaic acid, kojic acid, resveratrol, trans-retinoic acid, glutathione, vitamin C, vitamin E, N-acetyl-4S-cysteaminylphenol, glabridine, diethyl trioxopimelate, thioctic acid, alpha-hydroxyacids, *Morus alba* extract, *Thea sinensis* extract, *Chamomilla recutica* extract, *Ascophylum nodosum* extract, and mixtures thereof.

The amount of the second depigmenting agent in the cosmetic composition of the invention will vary depending on the nature of said second depigment agent. For example, it is known that the effective amount of kojic acid, azelaic acid or trans-retinoic acid to induce depigmentation of the skin is respectively 1%, 20% or 0.1%.

As such, the cosmetic composition may comprise 0.01% to 25%, in particular 1% to 10%, more particularly 2% to 5%, by weight of the second depigmenting agent based on the weight of the composition.

According to an embodiment of the present invention, the cosmetic composition may further comprise an adjuvant selected in the group consisting of a thickener, a water-soluble colorant, a perfume, a vitamin, a surfactant, a preservative, a bactericide, and mixtures thereof. Such cosmetic ingredients commonly used in the skin care industry, which are suitable for use in the compositions of the present invention, are e.g. described in the CTFA Cosmetic Ingredient Handbook, Second Edition (1992). The necessary amounts of the cosmetic and dermatological adjuvants and additives can easily be chosen by a skilled person in this field depending on the desired properties of the resulting composition.

Examples of suitable thickeners that may be introduced in the composition of the present invention include gum arabic, ghatti gum, karaya gum, carob gum, guar gum, tamarind gum, xanthan gum, gellan, pectins, tragacanth, agar, alginates, carrageenan, furcelleran, konjac; cellulose derivatives, such as hydroxyethylcellulose, hydroxypropylcellulose, hydroxypropylmethylcellulose, methylcellulose, ethylhydroxyethylcellulose, carboxymethylcellulose and the quaternized derivatives of cellulose; and mixtures thereof.

Examples of suitable water-soluble colorants that may be introduced in the composition of the present invention include organic colorants selected from natural colorants of animal origin such as carminic acid (Carmine or Natural Red 4), colouring material of vegetable origin such as bixin or Natural Orange 4, norbixin, betanin; anthocyans, chlorophyllins, and caramel; synthetic colorants such as molecules possessing at least one group selected from "nitroso" groups (Acid Green 1 etc.), "nitro" groups (Ext D&C Yellow 7, etc.), "azo" groups (Pigment Red 4, Solvent Orange 1, Solvent Red 3, Solvent Red 23, Pigment Red 57:1, Food Red 1, Acid Red 14, Acid Orange 7, FD&C Yellow 6, FD&C Red 40, D&C Red 33, FD&C Yellow 5 etc.), "xanthene" groups (D&C Yellow 8, D&C Orange 5, D&C Red 21, D&C Red 27, FD&C Red 3), "quinoline" groups (D&C Yellow 10 etc.), "anthraquinone" groups (Ext D&C Violet 2, D&C Green 5 etc.), "indigoid" groups (FD&C Blue 2, D&C Red 30 etc.), "cyanin" groups, in particular the phthalocyanins (Pigment Blue CI 77160 etc.), "triarylmethane" groups; and mixtures thereof.

Examples of suitable perfumes that may be introduced in the composition of the present invention include essential oils, myrcene, dihydromyrcenol, citral, tagetone, cis-geranic acid, citronellic acid, limonene, linalool and mixtures

thereof.

Examples of suitable vitamins that may be introduced in the composition of the present invention include vitamin B1 (thiamine), vitamin B2 (riboflavin), vitamin B3 (nicotinic acid), also known as vitamin PP (Pellagra Preventive), vitamin B5 (pantothenic acid), vitamin B6 (pyridoxine), vitamin B8 or H (biotin), vitamin B9 (folic acid), vitamin B12 (cobalamin), vitamin A (retinol), vitamin D3 (cholecalciferol), vitamin K (phylloquinone), and mixtures thereof.

The surfactant that may be introduced in the cosmetic composition of the present invention may be a nonionic, anionic, cationic or amphoteric surfactant. Examples of suitable surfactants include fatty alcohols such as cetyl alcohol, stearyl alcohol, cetostearyl alcohol, oleyl alcohol; ethoxylated and/or propoxylated fatty alcohols; ethers of fatty alcohols and of polyethylene glycol or a glucoside; fatty esters obtained by reaction of a fatty acid and a polyol such as glycerol, sorbitan, sucrose, polyethylene glycol, polypropylene glycol, 1,1,1-trimethylolpropane, pentaerythritol; ethoxylated and/or propoxylated fatty esters; silicone surfactants such as cetyldimethicone copolyol and modified polyether polysiloxanes; betaine and its derivatives; polyquaterniums; ethoxylated fatty alcohol sulphate salts; sulphosuccinates; sarcosinates; alkyl- and dialkyl phosphates and their salts such as potassium cetyl phosphate; soaps of fatty acids; and mixtures thereof.

Examples of suitable preservatives and bactericides that may be introduced in the composition of the present invention include benzoic acid, propionic acid, salicylic acid, sorbic acid, 4-hydroxybenzoic acid, pyrithione zinc, triclosan, chlorobutanol, chlorocresol, triclocarban, 2-phenoxyethanol, 2-bromo-2-nitropropane-1,3-diol, inorganic sulfites and bisulfites, salts thereof, esters thereof, and mixtures thereof.

The cosmetic composition may comprise 0.1% to 20%, in particular 1% to 10%, more particularly 2% to 5%, by weight of the adjuvant based on the weight of the composition.

Additionally, the cosmetic composition of the present invention may further comprise a photostabilizer or a mixture thereof. Photostabilizers are indeed useful to prevent the photodegradation of the at least one UV-filter that is optionally introduced in the composition of the present invention. More particularly, photostabilizers are able to prevent the formation of reactive intermediates of photo-unstable UV-filters. Indeed, these photoproducts may be toxic and may, for example lead to allergic reactions or contact dermatitis when they come into direct contact with the skin.

Examples of suitable photostabilizers that may be introduced in the composition of the present invention include 3,3-diphenylacrylate derivatives such as e.g. octocrylene (PARSOL® 340) or polyester-8 (Polycrylene®) or methoxycrylene (Solastay® S1); benzylidene camphor derivatives such as e.g. 4-methyl benzylidene camphor (PARSOL® 5000); benzalmalonate derivatives such as e.g. polysilicones-15 (PARSOL® SLX) or diethylhexyl syringylidene malonate (Oxynex® ST liquid); dialkyl naphthalates such as diethylhexyl naphthalate (Corapan® TQ), 2,4-pentanedione derivatives such as e.g. trimethoxybenzylidene pentanedione (Synoxyl® HSS), and mixtures thereof.

The cosmetic composition according to the invention may be formulated in any suitable form for topical administration. For example, the composition may be a solution, such as an aqueous, aqueous-alcoholic or oily solution; a dispersion, such as a lotion or serum; an emulsion, such as an oil-in-water emulsion, a water-in-oil emulsion, a multiple phase emulsion (for example an oil-in-water-in-oil emulsion or a water-in-oil-in-water emulsion) or a microemulsion; a gel, such as an aqueous or lipophilic gel; or a vesicular dispersion, such as an ionic and/or nonionic vesicular dispersion. These compositions are prepared by the usual methods known to one skilled in the art.

The invention will be described in greater detail with the aid of the examples that follow, which are given for purely illustrative purposes.

## EXAMPLES

### Example 1: Synthesis of umbelliprenin

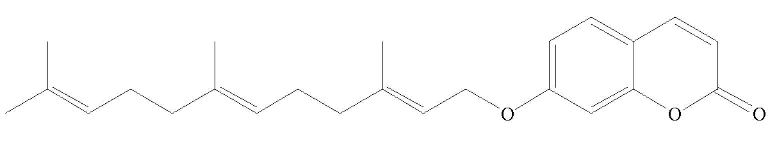

A mixture of 7-hydroxycoumarin (5.0 mmol), *trans-trans* farnesyl bromide (4.8 mmol) and dry potassium carbonate (6.0 mmol) dissolved in acetone (10 ml) was heated at 80 °C for 1 hour under magnetic strirring, cooled and then poured into water (100 ml). The resulting solution was extracted with *n*-hexane (3 x 20 ml). The combined organic phases were dried over anhydrous sodium sulphate, filtered and concentrated to dryness under vacuum. The resulting brownish solid was purified by crystallization (MeOH) affording umbelliprenin as a white solid in 74 % yield.
Melting point: 61-63°C
$^1$H NMR (200 MHz, $CDCl_3$) $\delta$ 7.60 (d, $J$ = 9.45 Hz, 1H), 7.32 (d, $J$ = 8.52 Hz, 1H), 6.84 (dd, $J_1$ = 8.52 Hz, $J_2$ = 2.03 Hz, 1H), 6.77 (d, $J$ = 2.03 Hz, 1H), 6.24 (d, $J$ = 9.5 Hz, 1H), 5.42 (d, $J$ = 7.02 Hz, 1H), 5.08-5.05 (m, 2H), 4.56 (d, $J$ = 7.02

Hz, 2H), 2.09-2.06 (m, 4H), 2.04-2.01 (M, 2H), 1.95 (t, *J* = 8.04 Hz, 2H), 1.77 (s, 3H), 1.64 (s, 3H), 1.56 (s, 3H)
$^{13}C$ NMR (50.1 MHz, $CDCl_3$) 5 162.0, 161.0, 155.6, 143.3, 142.3, 135.7, 131.0, 128.5, 124.3, 123.6, 118.4, 113.0, 112.7, 112.2, 101.3, 65.4, 39.4, 26.5, 26.0, 25.8, 17.5, 16.8, 15.9.
EIMS m/z 366 $[C_{24}H_{30}O_3]^+$ (4.6)

**Example 2: Synthesis of tesmilifene**

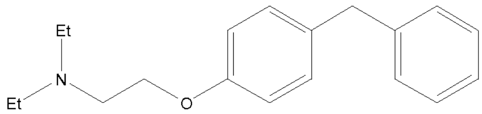

The procedure was previously described in « Poirot M. et al., Bioorganic & Medicinal Chemistry, 2000, 8, p. 2007-2016 »
2-diethylaminoethylchloride (0.50 g, 2.72 mmol) was added to a solution of 4-benzylphenol (0.58 g, 2.72 mmol) and $K_2CO_3$ (0.69 g, 5.44 mmol) in 15 mL of a mixture of dry DMF:acetone (1:1). The reaction mixture was maintained for 18 h at 60°C. The mixture was then filtered and poured into 100 mL of cold water and extracted twice with 100 mL of diethyl ether. The organic layer was washed 3 times with 10 mL of an aqueous solution of sodium hydroxide (0.1 N) and then 3 times with 10 mL of brine. The ethereal organic layer was then extracted with 5 mL of hydrochloric acid (12 N). The aqueous layer was collected and concentrated. The white solid was then recrystallized in a mixture of isopropanol:acetone (3:1) to give 0.78 g (Yield=90%) of white crystals as the hydrochloride salt and exhibits the following characteristics:
Melting point: 156-158°C;
$^1H$ NMR, $\delta$ (DMSO-d6): 7.1-7.6 (m, 9H, ArH), 3.91 (s, 2H, $ArCH_2$), 4.4 (t, J=5.8Hz, 2H, $ArOCH_2$), 2.82 (t, J=5.8Hz, 2H, $CH_2N$), 2.63 (q, 4H, $NCH_2$), 1.2 (t, 6H, $CH_3$);
Mass Spectrometry: m/z=283 (M+1).

**Example 3: Synthesis of PBPE**

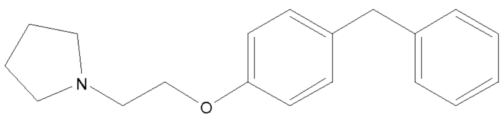

The procedure was previously described in « Poirot M. et al., Bioorganic & Medicinal Chemistry, 2000, 8, p. 2007-2016 »

- Synthesis of 2-(4-Benzyl-phenoxy)-ethanol

The same procedure was used as for tesmilifene prepared in example 2 using 2-chloroethanol instead of 2-diethylaminoethylchloride. The reaction mixture was maintained for 18 h at 60°C. The mixture was then filtered, poured into 100 mL of cold water and extracted twice with 100 mL of diethyl ether. The organic layer was washed 3 times with 10 mL of an aqueous solution of sodium hydroxide (0.1 N) and then 3 times with 10 mL of brine. The organic layer was concentrated under reduced pressure and the white solid was then recrystallized in hexane to give the desired product with a yield of 85%. The title compound exhibits the following characteristics:
Melting point: 70-72°C;
$^1H$ NMR $\delta$ (DMSO-d6): 7.1-7.6 (m, 9H, ArH), 4.01 (m, 4H, $OCH_2CH_2O$), 3.90 (s, 2H, $ArCH_2$);
Mass Spectrometry: m/z=228 (M+).

- Synthesis of 2-(4-Benzyl-phenoxy)-ethylbromine

2-(4-Benzyl-phenoxy)-ethanol (2 g, 8.8 mmol) of was dissolved in 20 mL of $CH_2Cl_2$ containing 1.1 equivalents of $CBr_4$ (1.3 g, 13.1 mmol) at 0°C. 1.2 equivalents of triphenylphosphine were then added. The mixture was stirred for 1 h at 0°C and then 1 h at room temperature. The solution was extracted 3 times with water, dried over $MgSO_4$, filtered and concentrated. The residue was recrystallized in a minimum of methanol to give 2.05 g (Yield=69%) of a white solid

having the following characteristics:
Melting point: 60-62°C;
$^1H$ NMR δ (DMSO-d6): 7.1-7.6 (m, 9H, ArH), 4.28 (t, 2H, $ArOCH_2$), 3.91 (s, 2H, $ArCH_2$), 3.62 (t, 2H, $CH_2Br$);
Mass Spectrometry: m/z=291 (M+).

- Synthesis of PBPE

A solution 0.5 g of compound 2-(4-Benzyl-phenoxy)-ethylbromine (1.7 mmol) and 1.1 equivalents of pyrrolidine in 15 mL of dry DMF were mixed at 60°C for 12 h. The mixture was poured into 100 mL of water and extracted twice with 25 mL of $CH_2Cl_2$. The organic layer was washed three times with 15 mL of NaOH (0.1 N), then three times with 15 mL of brine. The organic layer was dried over $MgSO_4$, filtered and concentrated to give 0.58 g (Yield=95%) of a yellow liquid having the following characteristics:
1H NMR δ (DMSO-d6): 6.6-7.6 (m, 11H, ArH + pyrrolidine α-H), 6.2 (m, 2H pyrrolidine β-H), 3.91 (s, 2H, $ArCH_2$), 4.06 (t, J=5.8 Hz, 2H, $ArOCH_2$), 4.18 (t, J=5.8 Hz, 2H, $CH_2N$);
Mass Spectrometry: m/z=278 (M+).

**Example 4: Effect of umbelliprenin on melanin content in normal murine melanocytes**

Melanin content was measured using a previously reported procedure (Ando H, J. Lipid Res., 1999, 40, p. 1312). Normal murine melanocytes were seeded and treated for 48 hours with increasing doses of umbelliprenin, 100μM of kojic acid (KA) or carrier solvent (DMSO). $5.10^6$ cells were centrifuged at 1500 rpm for 5 minutes at 4°C. The cell pellet was washed twice with phosphate buffer saline, transferred in an Eppendorf vial and centrifuged at 5000 g for 5 minutes at 4°C. The supernatant was discarded. 200 μL of water and 1 mL of EtOH/Ether (1/1) were added to precipitate melanin. The mixture was incubated for 15 minutes at room temperature, centrifuged at 5000 g for 5 minutes and the supernatant was discarded. The precipitate was solubilized by 300 μL of a mixture of aqueous sodium hydroxide (1 M)/DMSO 90/10 after heating at 80°C for 1 hour. The absorbance was measured at 405 nm. The melanin content was expressed as a percentage of control (=100%).

The results are shown in Figure 1. Umbelliprenin significantly decreases melanin content in a dose dependent manner. The diminution of melanin content was higher for umbelliprenin compared with kojic acid, a commercially available whitening compound. This example demonstrates that umbelliprenin has whitening properties.

**Example 5: Effect of PBPE and tesmilifene on melanin content in normal murine melanocytes**

Normal murine melanocytes were seeded and treated for 48 hours with increasing doses of PBPE, tesmilifene (Tes) or carrier solvent (ethanol). Melanin content was measured as described in example 4.

The results are shown in Figure 2. Both PBPE and tesmilifene significantly decrease melanin content in a dose dependent manner. This example demonstrates that PBPE and tesmilifene have whitening properties.

**Example 6: Umbelliprenin, PBPE and tesmilifene decrease melanin content in normal murine melanocytes treated with alpha melanocyte stimulating hormone (a-MSH).**

Normal murine melanocytes were seeded and treated for 48 hours with α-MSH (100 nM) in association with PBPE (20 μM), tesmilifene (10 μM), umbelliprenin (40 μM) or carrier solvent (DMSO for umbelliprenin and ethanol for tesmilifene or PBPE). Melanin content was measured as described in example 4. The results are shown in Figure 3. α-MSH, a physiological inducer of melanogenesis, triggers an increase of melanin content in normal murine melanocytes whereas the addition of umbelliprenin, PBPE and tesmilifene inhibits the melanogenic effect of α-MSH.

**Example 7: Effect of umbelliprenin, PBPE and tesmilifene on melanin content in normal murine melanocytes exposed to ultraviolet (UV-B) irradiation.**

Normal murine melanocytes were seeded and exposed to ultraviolet (UV-B) irradiation (20 $mJ/cm^2$) using RMX 3W Vilber Lourmat Biospectre according to the manufacturer's instructions and then treated with PBPE (20 μM), tesmilifene (10 μM), umbelliprenin (40 μM) or carrier solvent (DMSO for umbelliprenin and ethanol for tesmilifene or PBPE). Melanin content was measured as described in example 6. The results are shown in Figure 4. UV-B irradiation induces an increase of melanin content in normal murine melanocytes whereas the addition of umbelliprenin, PBPE and tesmilifene inhibits the melanogenic effect of UV-B.

**Example 8: Effect of umbelliprenin, PBPE and tesmilifene combinations on melanin content in normal murine melanocytes**

Normal murine melanocytes were seeded and treated for 48 hours with fixed doses of PBPE, tesmilifene, umbelliprenin or a combination of those, or carrier solvent (DMSO for umbelliprenin, ethanol for tesmilifene or PBPE and EtOH/DMSO 1/1 for the combination). Melanin content was measured as described in example 4.

The results are shown in Figure 5. Combination treatments significantly decrease melanin level with higher efficacy compared to umbelliprenin, PBPE or tesmilifene alone. This example demonstrates that molecules of general formula I can be combined to achieve optimized whitening properties.

**Example 9: Effect of umbelliprenin, PBPE and tesmilifene on tyrosinase activity in normal murine melanocytes.**

Tyrosinase activity was measured using a previously reported procedure (Ando H, J. Lipid Res., 1999, 40, p. 1312). Normal murine melanocytes were seeded and treated for 48 hours with kojic acid (100 μM), PBPE (20 μM), tesmilifene (10 μM), umbelliprenin (40 μM) or carrier solvent (DMSO for umbelliprenin and ethanol for tesmilifene or PBPE). $5.10^6$ cells were centrifuged at 1500 rpm for 5 minutes at 4°C. The cell pellet was washed twice with phosphate buffer saline, transferred in an Eppendorf vial and centrifuged at 5000 g for 5 minutes at 4°C. The supernatant was discarded. Cell lysis was performed with an aqueous solution of sodium deoxycholate 0.5% (1 mL per cell pellet). The suspension was incubated at 0°C for 15 minutes and then 3 mL of a freshly prepared solution of L-DOPA (0.1% w/w) in phosphate buffer at 0.1 M (pH 6.8) was added to the cell lysate. The mixture was incubated at 37°C for 10 min. Tyrosinase activity was measured with a spectrophotometer to quantify the oxidation of L-DOPA to DOPAchrome. The absorbance was measured at 475 nm. The tyrosinase activity was expressed as a percentage of control (=100%). Control corresponds to the tyrosinase activity measured in the cell lysate from normal murine melanocytes treated with carrier solvent. Melanin content of treated murine melanocytes was also measured as described in example 4.

The results are shown in Figure 6. Umbelliprenin, PBPE and tesmilifene decrease tyrosinase activity levels in normal murine melanocytes. The diminution of melanin content was higher for umbelliprenin, tesmilifene and PBPE compared with kojic acid, a commercially available whitening compound.

**Example 10: Whitening effect of umbelliprenin on normal murine melanocytes pellet.**

Normal murine melanocytes were seeded and treated for 48 hours with umbelliprenin (40 μM) or carrier solvent (DMSO). Cells were detached by trypsinization, collected in phosphate buffer saline and centrifuged at 1500 rpm for 5 minutes at 4°C. Cells pellet was washed with phosphate buffer saline, centrifuged and the supernatant was discarded. Cell pellets were then photographed.

The results are shown in Figure 7. The appearance of melanocytes treated with umbelliprenin was much lighter than melanocytes treated with the carrier solvent.

**Example 11: Effect of umbelliprenin, tesmilifene and PBPE on the expression of genes encoding melanogenic proteins in normal murine melanocytes**

Normal murine melanocytes were seeded and treated as described in example 9. We then performed RNA extraction. We added Trizol® (1 ml) on treated cells. After incubation (5 minutes at room temperature), we transferred the cells suspension into Eppendorf vial. Chloroform (200 μl at 4°C) was added, the mixture was manually stirred for 15 seconds, incubated for 5 minutes at room temperature and then centrifuged at 16000 g for 10 minutes at 4°C. Aqueous colorless layer was collected and isopropanol (500 μL at -20°C) was added. The mixture was manually stirred for 15 seconds, incubated for 15 minutes at room temperature and the centrifuged at 16200 g for 10 minutes at 4°C. The supernatant was discarded and the precipitate was washed with 1 mL of $EtOH/H_20$ (3/1) at -20°C. The mixture was centrifuged at 16000 g for 10 minutes at 4°C, the supernatant discarded and the pellet was allowed to dry for 10 minutes at room temperature. The pellet was dissolved in RNAse-free water (30 μL). The amount and purity of RNA was determined by measuring the absorbance at 230, 260 and 280 nm with a nanodrop ND-1000 spectrophotometer (Thermo Scientific) according to the manufacturer's instructions.

We then performed RT-qPCR experiment. We prepared a mixture containing RNA (1 μg), reverse transcriptase (1 μL), the reaction mixture « iScript®5x » (4 μL) and RNAse-free water (qsp 20 μl). The mixture was vortexed, centrifuged for few seconds and then introduced into a thermocycler. Reverse transcription was performed using the following program : 5 min at 25°C, 30 min at 42°C and then 5 min at 85°C. Eppendorf vial was maintained at 4°C until the beginning of the qPCR experiment. We then examined the expression of melanogenic genes (Tyrosinase, Mitf, TRP-1 and TRP-2) and housekeeping genes (glyceraldehyde 3-phosphate dehydrogenase and Cyclophiline A1) by qPCR. For this purpose, cDNA was diluted with RNAse-free water (180 μL) and then we prepared a mixture containing: primers, Syber

Green® and RNAse-free water. We mixed in a 96-well plate cDNA (5 μL) and the mixture (20 μL). The plate was sealed with an adhesive sealing film, centrifuged at 4000 rpm for 3 minutes and then introduced in a thermocycler. The following program was used to amplify cDNA of the studied genes: 95°C for 3 min followed by 50 amplification cycles (15 sec at 95°C and then 1 minute at 60°C). At the end of the program, melting curves were generated (95°C for 1 minute and 80 cycles of 10 sec at 95°C).

The results are shown in figure 8. Umbelliprenin significantly repressed the expression of melanogenic genes whereas this effect was not observed with tesmilifene and PBPE. This experiment showed that whitening property of umbelliprenin is associated with a blockage of melanogenic proteins expression at the transcriptional level whereas the depigmenting activity of PBPE and tesmilifene does not involve this process.

**Example 12: Cytotoxicity of umbelliprenin, tesmilifene and PBPE**

Normal murine melanocytes were seeded and treated for 24, 48 and 72 hours with PBPE (20 μM), tesmilifene (10 μM), umbelliprenin (40 μM) or carrier solvent (DMSO for umbelliprenin and ethanol for tesmilifene or PBPE). Cells were detached by trypsinization, collected in phosphate buffer saline and centrifuged at 1500 rpm for 5 minutes at 4°C. Cells pellets were resuspended in the trypan blue solution (0.25%, w/v in PBS) and counted in a Malassez cell under a light microscope. The percentage of cell viability was calculated using the following formula:

% cell viability= [1-(blue cells/total cells)] x100

The results are shown in figure 9. Umbelliprenin, tesmilifene and PBPE have no significant impact on cell viability at doses that are effective to stimulate whitening of normal murine melanocytes.

## Claims

1. Method for lightening a skin or a mucous membrane in a patient, said method comprising topically administering to said patient an effective amount of a depigmenting agent of general formula (I):

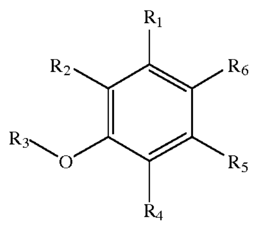

(I)

wherein

$R_1$ is H or hydroxy;
$R_2$ is H or methoxy;
$R_3$ is $C_5$-$C_{15}$ alkenyl optionally substituted by hydroxy or $R_3$ is $-CH_2-CH_2-NR_7R_8$;
$R_4$ is H or methoxy;
$R_5$ is H or hydroxy;
$R_6$ is selected in the group consisting of $-CH_2-Ph$; -COOH; -CH=CH-COOH; -CH=CH-CHO;$-CH=CH-CH_2OH$; -CO-CH=CH-Ph; wherein Ph is a phenyl optionally substituted by hydroxy; $-CH_2CH_2COOH$; $-(CH_2)_n-OH$ with n=1, 2 or 3;
or $R_5$ and $R_6$ form, with the carbon atoms to which they are attached, an unsubstituted fused 5,6-dihydropyran-2-one ring or an optionally substituted fused 2,3-dihydro-1,4-naphthalenedione system;
$R_7$ and $R_8$ are each independently $C_1$-$C_3$ alkyl or $R_7$ and $R_8$ form, with the nitrogen atom to which they are attached, a five-membered ring.

2. Method according to claim 1, wherein the depigmenting agent corresponds to general formula (I), wherein:

$R_1$ is H;

$R_2$ and $R_4$ are each independently H or methoxy;
$R_3$ is unsubstituted $C_5$-$C_{15}$ alkenyl;
$R_5$ is H or hydroxy; and
$R_6$ is selected in the group consisting of $-CH_2-Ph$; -COOH; -CH=CH-COOH; -CH=CH-CHO;$-CH=CH-CH_2OH$; -CO-CH=CH-Ph; wherein Ph is a phenyl optionally substituted by hydroxy; $-CH_2CH_2COOH$; $-(CH_2)_n-OH$ with n=1, 2 or 3.

**3.** Method according to claim 1, wherein the depigmenting agent corresponds to general formula (I), wherein:

$R_1$, $R_2$, $R_4$ and $R_5$ are H;
$R_3$ is $-CH_2-CH_2-NR_7R_8$;
$R_6$ is $-CH_2-Ph$; and
$R_7$ and $R_8$ are each independently $C_1$-$C_3$ alkyl or $R_7$ and $R_8$ form, with the nitrogen atom to which they are attached, a five-membered ring.

**4.** Method according to claim 1, wherein the depigmenting agent corresponds to general formula (I), wherein:

$R_1$, $R_2$ and $R_4$ are H;
$R_3$ is $C_5$-$C_{15}$ alkenyl optionally substituted by hydroxy;
$R_5$ and $R_6$ form, with the carbon atoms to which they are attached, an unsubstituted fused 5,6-dihydropyran-2-one ring.

**5.** Method according to claim 1, wherein the depigmenting agent corresponds to general formula (I), wherein:

$R_1$ is OH;
$R_2$ and $R_4$ are H;
$R_3$ is unsubstituted $C_5$-$C_{15}$ alkenyl;
$R_5$ and $R_6$ form, with the carbon atoms to which they are attached, an optionally substituted fused 2,3-dihydro-1,4-naphthalenedione system.

**6.** Method according to claim 1, wherein the depigmenting agent is selected in the group consisting of:

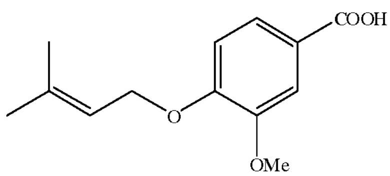 , 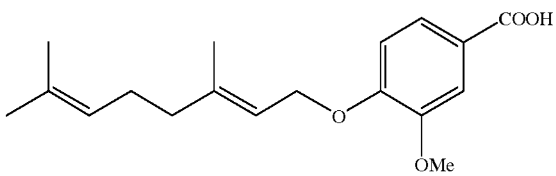 ,

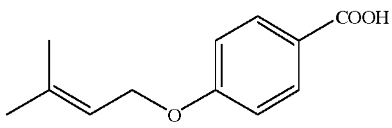 , 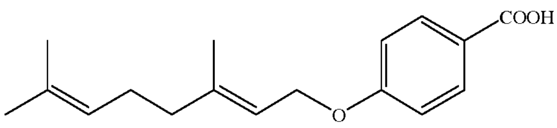 ,

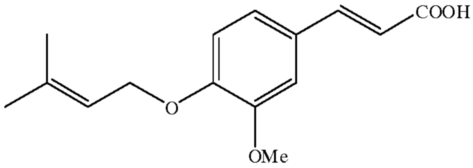 , 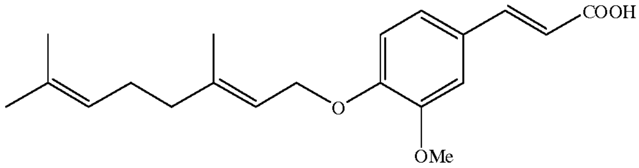 ,

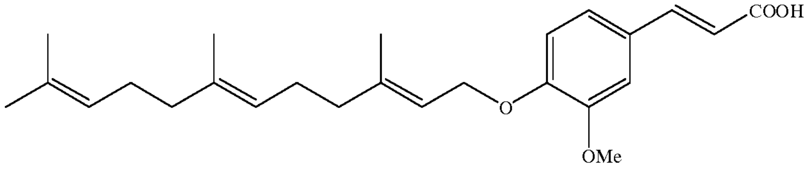 ,

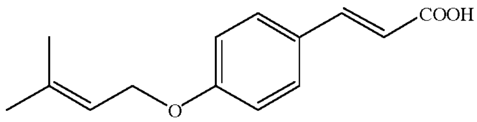,
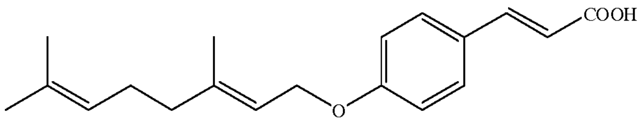,

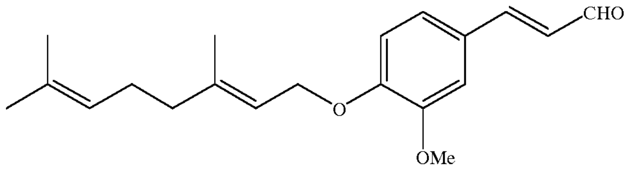,

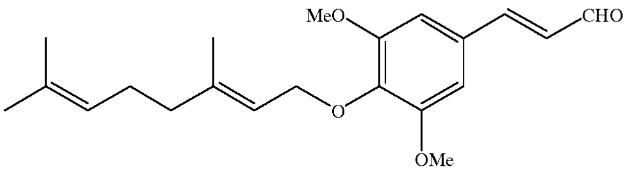,
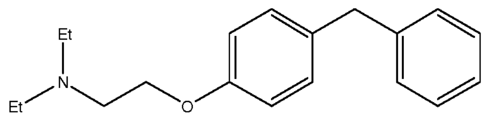,

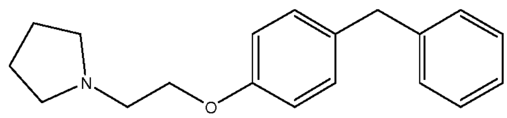,
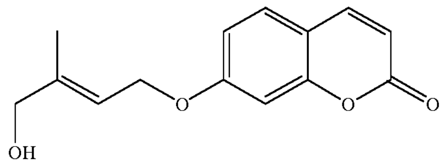,

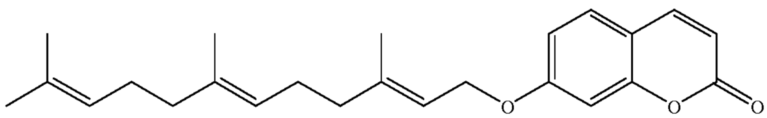,

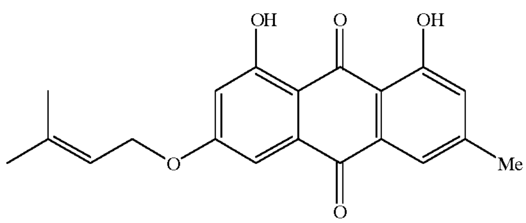,

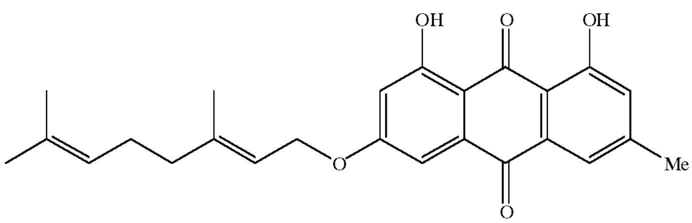,

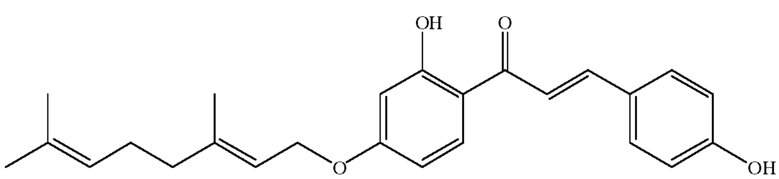,

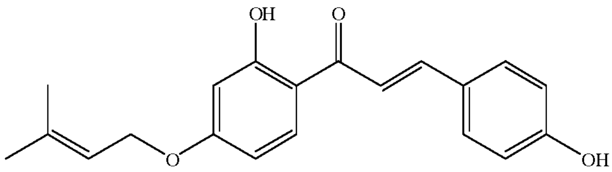,

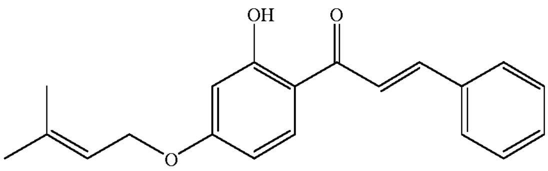, 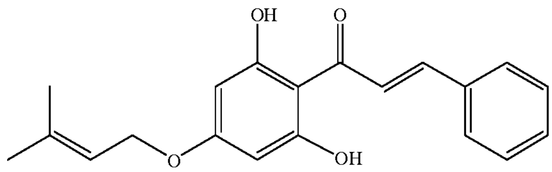,

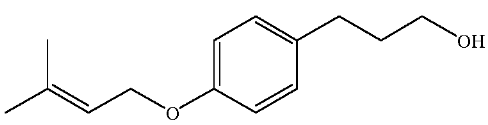, 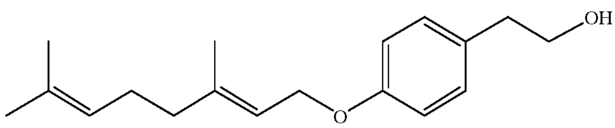,

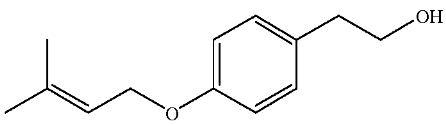, 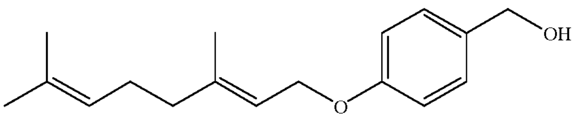,

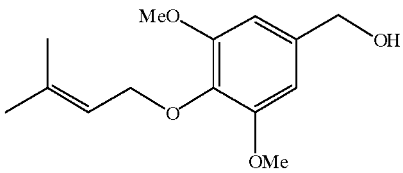, 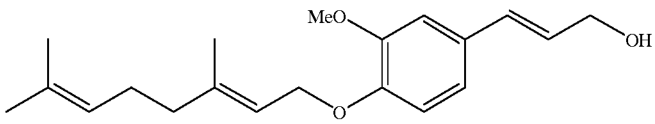,

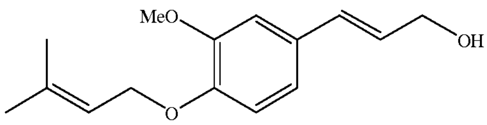, 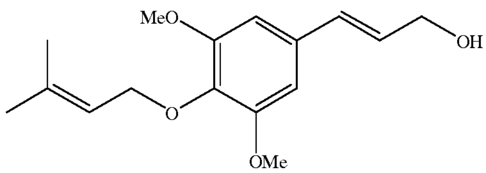,

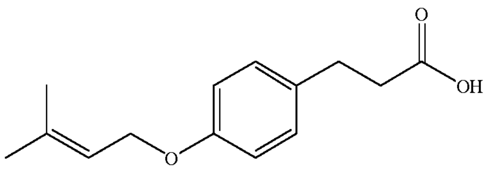,

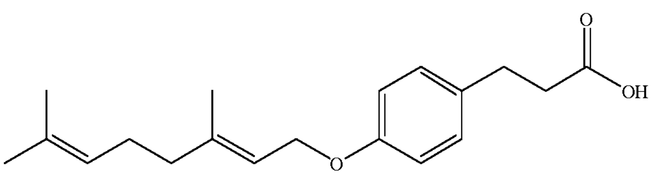, 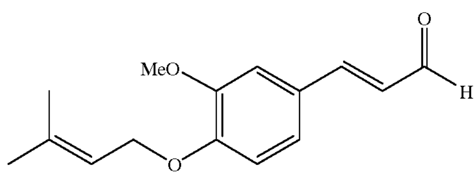,

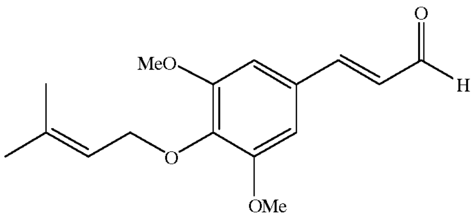
,
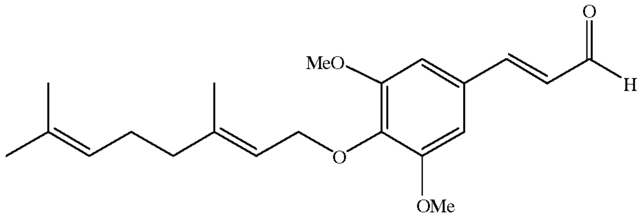
,

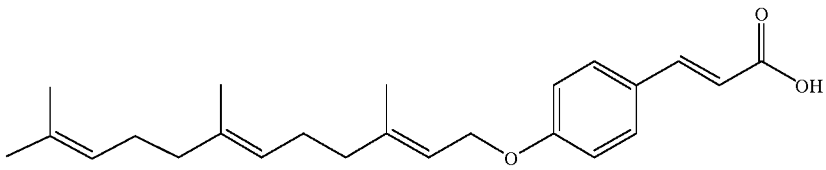
,

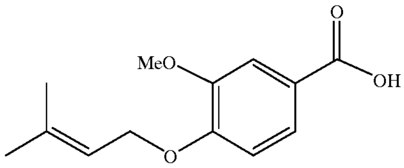
,
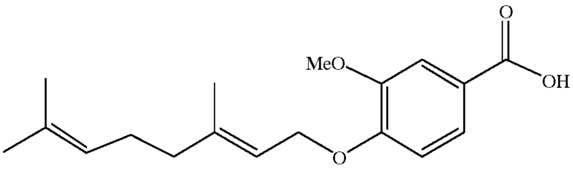
,

and mixtures thereof.

**7.** Cosmetic use of a depigmenting agent of general formula (I) as defined in any one of claims 1 to 6 for inducing lightening of a skin or a mucous membrane, improving the uniformity and/or clarity of a skin or a mucous membrane, and/or preventing, reducing and/or removing hyperpigmented spots.

**8.** Cosmetic composition comprising, in a cosmetically acceptable carrier, a depigmenting agent of general formula (I) as defined in any one of claims 1 to 6, a plant extract containing said depigmenting agent of general formula (I), and mixtures thereof,
wherein the cosmetic composition further comprises at least one UV-filter, with the proviso that said at least one UV-filter does not correspond to general formula (II)

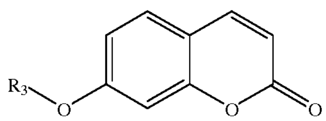

(II)

wherein $R_3$ is $C_5$-$C_{15}$ alkenyl optionally substituted by hydroxy.

**9.** Cosmetic composition according to claim 8, wherein said plant extract is selected in the group consisting of a plant extract from the genus *Ferula,* in particular *Ferula szowitsiana, Ferula sinkiangensis, Ferula assa-foetida, Ferula tunetana, Ferula flabelliloba, Ferula fukanensis, Ferula persica* and *Ferula aitchisonii;* a plant extract from the genus *Angelica,* in particular *Angelica sylvestris, Angelica komarovii, Angelica pubescens, Angelica pachycarpa, Angelica decursiva, Angelica archangelica* and *Angelica ursina;* a *Pimpinella anisum* extract; an *Anethum graveolens* extract; *a Ferulago campestris extract;* a *Cicuta virosa* extract; a *Seseli annuum* extract; a *Peucedanum zenkeri* seed extract; a *Thapsia villosa* fruit extract; a *Scandix pecten-veneris* extract; a *Scabiosa comosa* extract; a *Heracleum sphondylium* extract; an *Ammi majus* extract; a *Xanthogalum sachokianum* extract; a *Thamnosa montana* extract; a *Ligusticum seguieri* extract; and mixtures thereof.

**10.** Cosmetic composition according to any one of claim 8 or 9, wherein it comprises 0.0001% to 50%, in particular 0.1% to 20%, more particularly 1% to 10%, by weight of depigmenting agent of general formula (I) based on the weight of the composition.

**11.** Cosmetic composition according to any one of claims 8 to 10, wherein it further comprises a second depigmenting agent, or mixtures thereof.

**12.** Cosmetic composition according to claim 11, wherein the second depigmenting agent is selected in the group consisting of inhibitors of melanogenesis enzymes, copper chelating agents, antioxidants, inhibitors of melanosome transfer from melanocytes to keratinocytes, exfoliants, inhibitors of alpha-MSH production, inhibitors of endothelin production, inhibitors of adrenocorticotropic hormone (ATCH) production, and mixtures thereof.

**Patentansprüche**

**1.** Verfahren zum Aufhellen einer Haut oder einer Schleimhaut eines Patienten, wobei das Verfahren das topische Verabreichen einer wirksamen Menge eines Depigmentierungsmittels der allgemeinen Formel (I) an den Patienten umfasst:

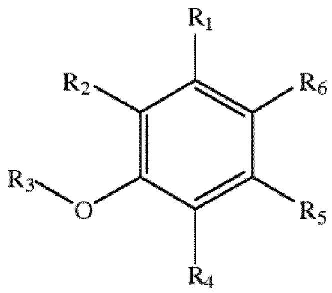

(I),

worin

$R_1$ H oder Hydroxy ist;
$R_2$ H oder Methoxy ist;
$R_3$ $C_5$-$C_{15}$-Alkenyl ist, gegebenenfalls substituiert durch Hydroxy, oder $R_3$ $-CH_2-CH_2-NR_7R_8$ ist;
$R_4$ H oder Methoxy ist;
$R_5$ H oder Hydroxy ist;
$R_6$ ausgewählt ist aus der Gruppe, bestehend aus $-CH_2-Ph$; -COOH; -CH=CH-COOH; -CH=CH-CHO; -CH=CH-$CH_2OH$; -CO-CH=CH-Ph; worin Ph ein Phenyl ist, das gegebenenfalls durch Hydroxy substituiert ist; $-CH_2CH_2COOH$; $-(CH_2)_n-OH$, wobei n = 1, 2 oder 3;
oder $R_5$ und $R_6$ mit den Kohlenstoffatomen, an die sie gebunden sind, einen unsubstituierten kondensierten 5,6-Dihydropyran-2-on-Ring oder ein gegebenenfalls substituiertes kondensiertes 2,3-Dihydro-1,4-Naphthalin-dion-System bilden;
$R_7$ und $R_8$ jeweils unabhängig voneinander $C_1$-$C_3$-Alkyl sind oder $R_7$ und $R_8$ mit dem Stickstoffatom, an das sie gebunden sind, einen fünfgliedrigen Ring. bilden

**2.** Verfahren nach Anspruch 1, worin das Depigmentierungsmittel der allgemeinen Formel (I) entspricht, worin:

$R_1$ H ist;
$R_2$ und $R_4$ jeweils unabhängig voneinander H oder Methoxy sind;
$R_3$ unsubstituiertes $C_5$-$C_{15}$-Alkenyl ist;
$R_5$ H oder Hydroxy ist; und
$R_6$ ausgewählt ist aus der Gruppe bestehend aus $-CH_2-Ph$; -COOH; -CH=CH-COOH; -CH=CH-CHO; CH=CH-$CH_2OH$; -CO-CH=CH-Ph; worin Ph ein Phenyl ist, das optional durch Hydroxy substituiert ist; $-CH_2CH_2COOH$; $-(CH_2)_n-OH$, wobei n = 1,2 oder 3.

**3.** Verfahren nach Anspruch 1, worin das Depigmentierungsmittel der allgemeinen Formel (I) entspricht, worin:

$R_1$, $R_2$, $R_4$ und $R_5$ H sind;
$R_3$ $-CH_2-CH_2-NR_7R_8$ ist;

$R_6$ $-CH_2-Ph$ ist; und
$R_7$ und $R_8$ jeweils unabhängig voneinander $C_1$-$C_3$-Alkyl sind oder $R_7$ und $R_8$ mit dem Stickstoffatom, an das sie gebunden sind, einen fünfgliedrigen Ring bilden.

**4.** Verfahren nach Anspruch 1, worin das Depigmentierungsmittel der allgemeinen Formel (I) entspricht, worin:

$R_1$, $R_2$ und $R_4$ H sind;
$R_3$ $C_5$-$C_{15}$-Alkenyl ist, das optional durch Hydroxy substituiert ist;
$R_5$ und $R_6$ mit den Kohlenstoffatomen, an die sie gebunden sind, einen unsubstituierten kondensierten 5,6-Dihydropyran-2-on-Ring bilden.

**5.** Verfahren nach Anspruch 1, worin das Depigmentierungsmittel der allgemeinen Formel (I) entspricht, worin:

$R_1$ OH ist;
$R_2$ und $R_4$ H sind;
$R_3$ unsubstituiertes $C_5$-$C_{15}$-Alkenyl ist;
$R_5$ und $R_6$ mit den Kohlenstoffatomen, an die sie gebunden sind, ein optional substituiertes kondensiertes 2,3-Dihydro-1,4-Naphthalindionsystem bilden.

**6.** Verfahren nach Anspruch 1, worin das Depigmentierungsmittel ausgewählt ist aus der Gruppe, bestehend aus:

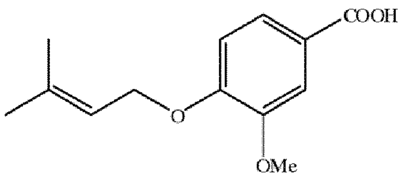
,
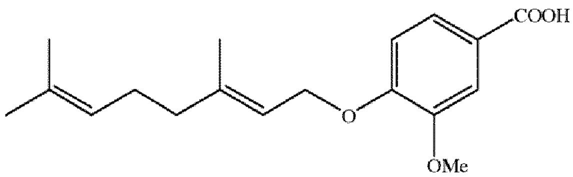
,

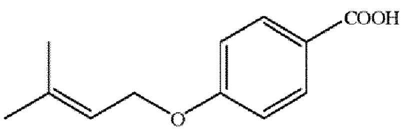
,
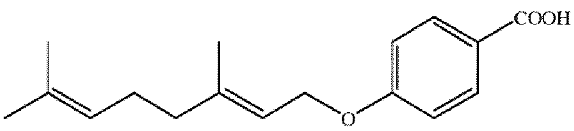
,

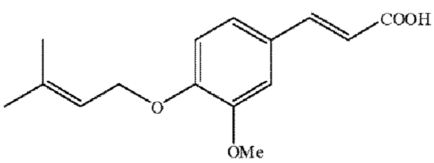
,
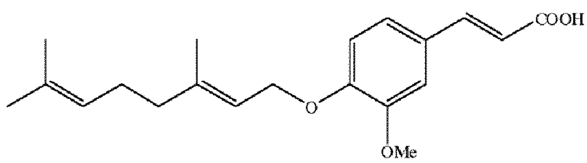
,

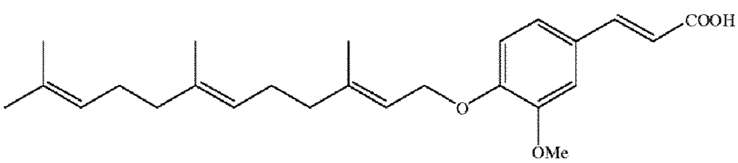
,

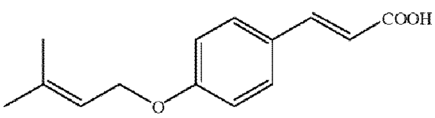
,
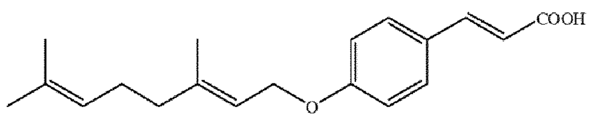
,

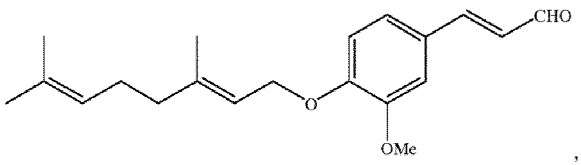,

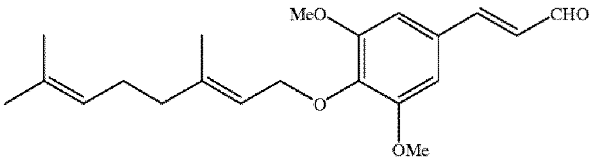, 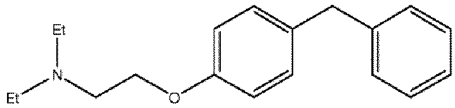,

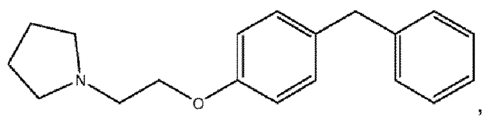, 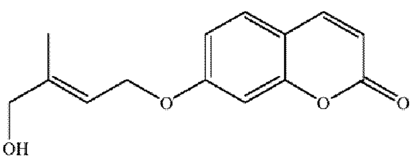,

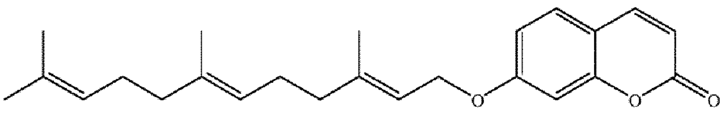,

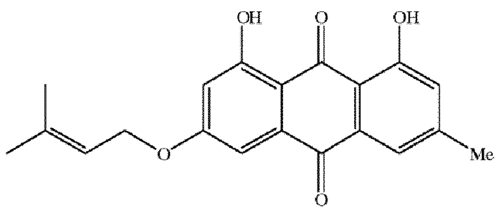,

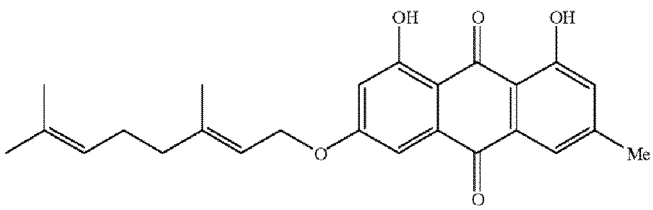,

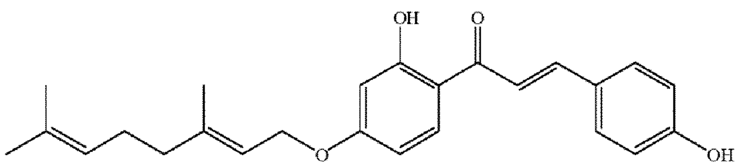,

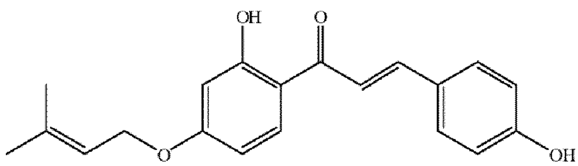,

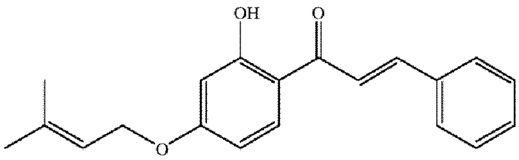 , 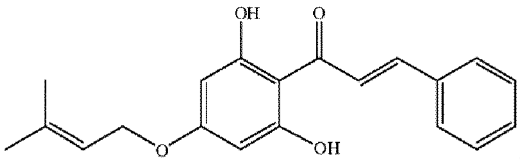 ,

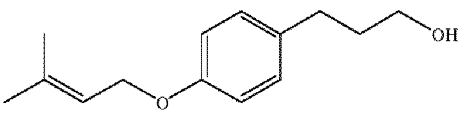 , 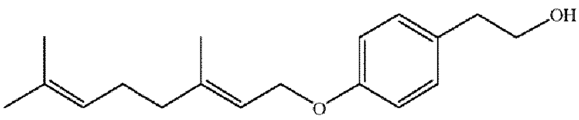 ,

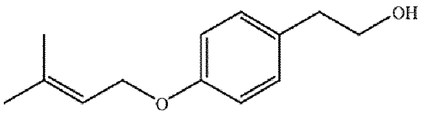 , 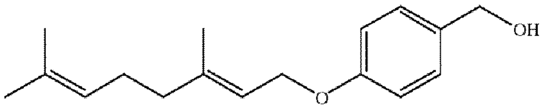 ,

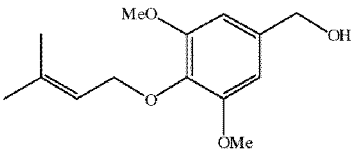 , 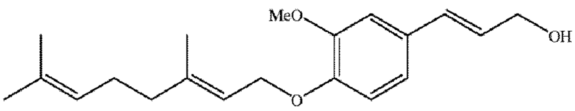 ,

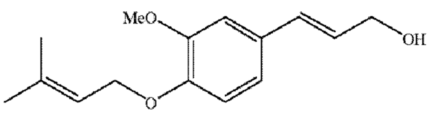 , 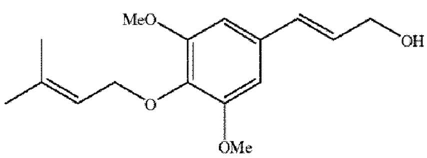 ,

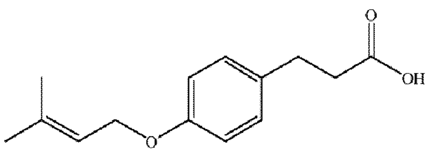 ,

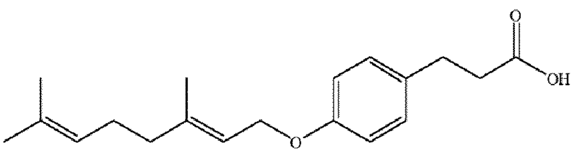 , 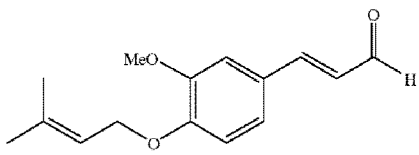 ,

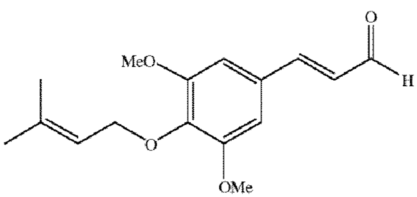 , 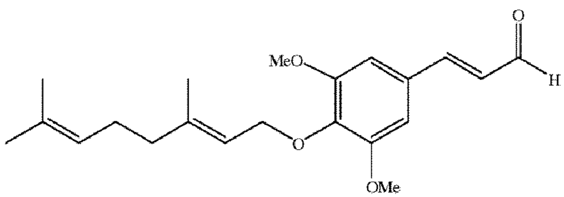 ,

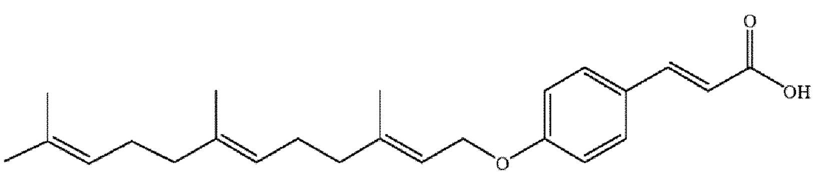 ,

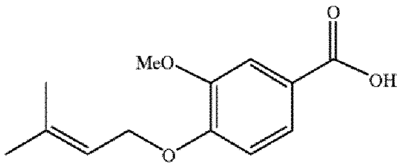
,
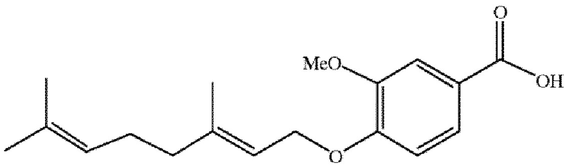
,

und Gemischen davon.

7. Kosmetische Verwendung eines Depigmentierungsmittels der allgemeinen Formel (I) nach einem der Ansprüche 1 bis 6 zur Induzierung der Aufhellung einer Haut oder einer Schleimhaut, zur Verbesserung der Gleichmäßigkeit und/oder Klarheit einer Haut oder einer Schleimhaut und/oder zur Vorbeugung, Reduzierung und/oder Entfernung von hyperpigmentierten Flecken.

8. Kosmetische Zusammensetzung, umfassend in einem kosmetisch verträglichen Träger ein Depigmentierungsmittel der allgemeinen Formel (I) nach einem der Ansprüche 1 bis 6, einen Pflanzenextrakt, der das Depigmentierungsmittel der allgemeinen Formel (I) enthält, und Gemische davon, worin die kosmetische Zusammensetzung weiterhin mindestens einen UV-Filter umfasst, mit der Maßgabe, dass der mindestens eine UV-Filter nicht der allgemeinen Formel (II) entspricht.

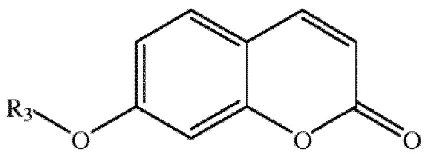

(II) ,

worin $R_3$ $C_5$-$C_{15}$-Alkenyl ist, das gegebenenfalls durch Hydroxy substituiert ist.

9. Kosmetische Zusammensetzung nach Anspruch 8, worin der Pflanzenextrakt ausgewählt ist aus der Gruppe bestehend aus einem Pflanzenextrakt aus der Gattung Ferula, insbesondere Ferula szowitsiana, Ferula sinkiangensis, Ferula assa-foetida, Ferula tunetana, Ferula flabelliloba, Ferula fukanensis, Ferula persica und Ferula aitchisonii; einem Pflanzenextrakt aus der Gattung Angelica, insbesondere Angelica sylvestris, Angelica komarovii, Angelica pubescens, Angelica pachycarpa, Angelica decursiva, Angelica archangelica und Angelica ursina; einem Pimpinella anisum-Eextrakt; einem Anethum graveolens-Extrakt; einem Ferulago campestris-Extrakt; einem Cicuta virosa-Extrakt; einem Seseli annuum-Extrakt; einem Peucedanum zenkeri-Samenextrakt; einem Thapsia villosa-Fruchtextrakt; einem Scandix pecten-veneris-Extrakt; einem Scabiosa comosa-Extrakt; einem Heracleum sphondylium-Extrakt; einen Ammi majus-Extrakt; einem Xanthogalum sachokianum-Extrakt; einem Thamnosa montana-Extrakt; einem Ligusticum seguieri-Extrakt; und Gemische davon.

10. Kosmetische Zusammensetzung nach einem der Ansprüche 8 oder 9, worin sie 0,0001% bis 50%, im Speziellen 0,1% bis 20%, im Spezielleren 1% bis 10%, bezogen auf das Gewicht des Depigmentierungsmittels der allgemeinen Formel (I), bezogen auf das Gewicht der Zusammensetzung, umfasst.

11. Kosmetische Zusammensetzung nach einem der Ansprüche 8 bis 10, worin sie weiterhin ein zweites Depigmentierungsmittel oder Gemische davon umfasst.

12. Kosmetische Zusammensetzung nach Anspruch 11, worin das zweite Depigmentierungsmittel ausgewählt ist aus der Gruppe bestehend aus Inhibitoren von Melanogeneseenzymen, Kupferchelatbildnern, Antioxidantien, Inhibitoren der Melanosomenübertragung von Melanozyten auf Keratinozyten, Exfoliationsmitteln, Inhibitoren der alpha-MSH-Produktion, Inhibitoren der Endothelinproduktion, Inhibitoren der Produktion von adrenocorticotropem Hormon (ATCH) und Gemischen davon.

## Revendications

**1.** Procédé d'éclaircissement d'une peau ou d'une membrane muqueuse chez un patient, ledit procédé comprenant l'administration par voie topique audit patient d'une quantité efficace d'un agent de dépigmentation de formule générale (I) :

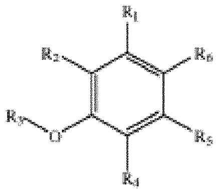

(I)

dans lequel

$R_1$ est un H ou un hydroxy ;
$R_2$ est un H ou un méthoxy ;
$R_3$ est un alcényle en $C_5$ à $C_{15}$ facultativement substitué par un hydroxy ou $R_3$ est un - $CH_2$-$CH_2$-$NR_7R_8$ ;
$R_4$ est un H ou un méthoxy ;
$R_5$ est un H ou un hydroxy ;
$R_6$ est choisi dans le groupe constitué de -$CH_2$-Ph ; -COOH ; -CH=CH-COOH ; - CH=CH-CHO ; -CH=CH-$CH_2$OH ; -CO-CH=CH-Ph ; dans lequel Ph est un phényle facultativement substitué par un hydroxy ; -$CH_2CH_2$COOH ; -$(CH_2)_n$-OH avec n = 1, 2 ou 3 ;
ou $R_5$ et $R_6$ forment, avec les atomes de carbone auxquels ils sont liés, un cycle 5,6-dihydropyran-2-one fusionné non substitué ou un système 2,3-dihydro-1,4-naphtalènedione fusionné facultativement substitué ;
$R_7$ et $R_8$ sont chacun indépendamment un alkyle en $C_1$ à $C_3$ ou $R_7$ et $R_8$ forment, avec l'atome d'azote auquel ils sont liés, un cycle à cinq chaînons.

**2.** Procédé selon la revendication 1, dans lequel l'agent de dépigmentation correspond à la formule générale (I), dans lequel :

$R_1$ est un H ;
$R_2$ et $R_4$ sont chacun indépendamment un H ou un méthoxy ;
$R_3$ est un alcényle en $C_5$ à $C_{15}$ non substitué ;
$R_5$ est un H ou un hydroxy ; et
$R_6$ est choisi dans le groupe constitué de -$CH_2$-Ph ; -COOH ; -CH=CH-COOH ; - CH=CH-CHO ; -CH=CH-$CH_2$OH ; -CO-CH=CH-Ph ; dans lequel Ph est un phényle facultativement substitué par un hydroxy ; -$CH_2CH_2$COOH ; -$(CH_2)_n$-OH avec n = 1, 2 ou 3.

**3.** Procédé selon la revendication 1, dans lequel l'agent de dépigmentation correspond à la formule générale (I), dans lequel :

$R_1$, $R_2$, $R_4$ et $R_5$ sont un H ;
$R_3$ est un -$CH_2$-$CH_2$-$NR_7R_8$ ;
$R_6$ est un -$CH_2$-Ph ; et
$R_7$ et $R_8$ sont chacun indépendamment un alkyle en $C_1$ à $C_3$ ou $R_7$ et $R_8$ forment, avec l'atome d'azote auquel ils sont liés, un cycle à cinq chaînons.

**4.** Procédé selon la revendication 1, dans lequel l'agent de dépigmentation correspond à la formule générale (I), dans lequel :

$R_1$, $R_2$ et $R_4$ sont un H ;
$R_3$ est un alcényle en $C_5$ à $C_{15}$ facultativement substitué par un hydroxy ;
$R_5$ et $R_6$ forment, avec les atomes de carbone auxquels ils sont liés, un cycle 5,6-dihydropyran-2-one fusionné non substitué.

**5.** Procédé selon la revendication 1, dans lequel l'agent de dépigmentation correspond à la formule générale (I), dans lequel :

$R_1$ est un OH ;
$R_2$ et $R_4$ sont un H ;
$R_3$ est un alcényle en $C_5$ à $C_{15}$ non substitué ;
$R_5$ et $R_6$ forment, avec les atomes de carbone auxquels ils sont liés, un système 2,3-dihydro-1,4-naphtalènedione fusionné facultativement substitué.

**6.** Procédé selon la revendication 1, dans lequel l'agent de dépigmentation est choisi dans le groupe constitué de :

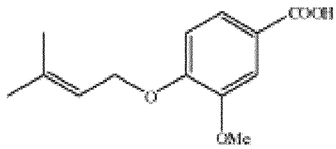 , 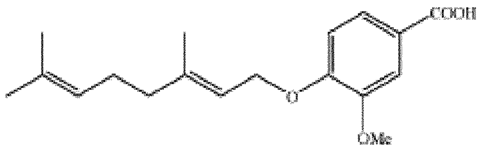 ,

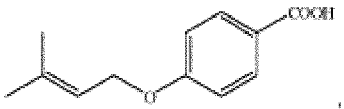 , 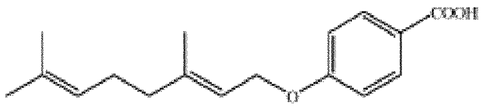 ,

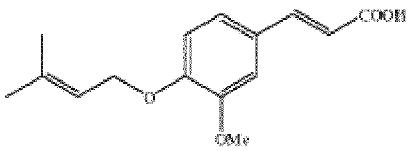 , 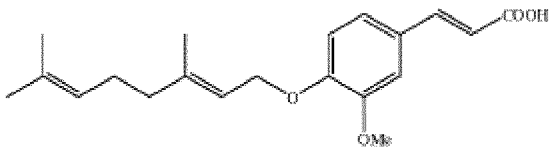 ,

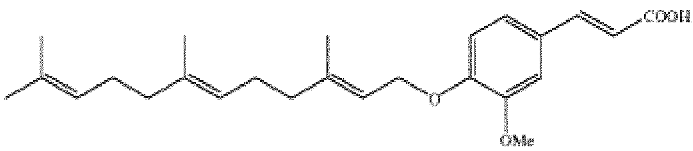 ,

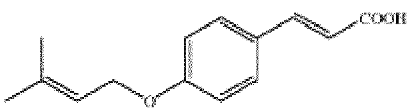 , 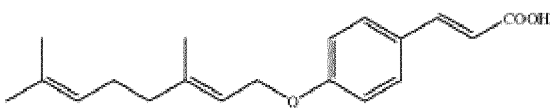 ,

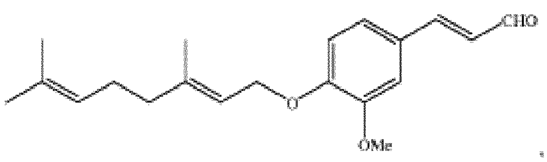 ,

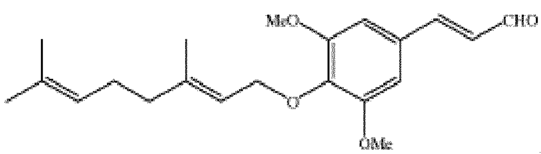 , 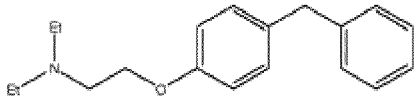 ,

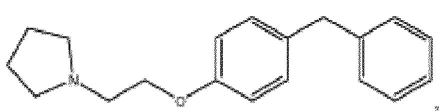 , 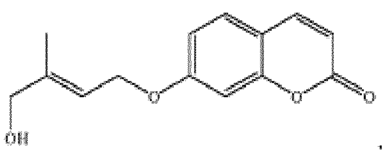 ,

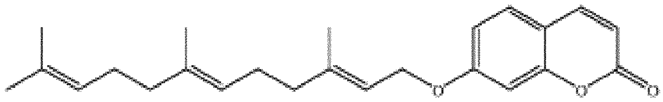,

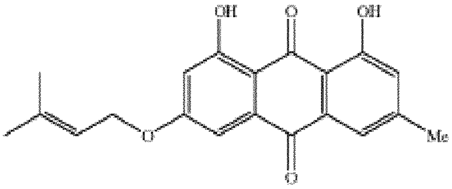,

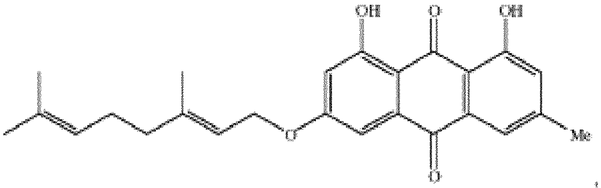,

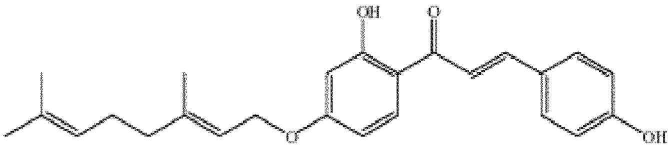,

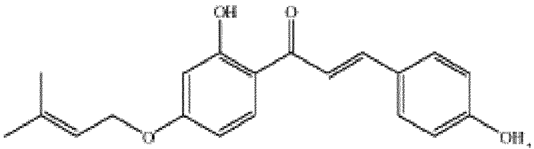,

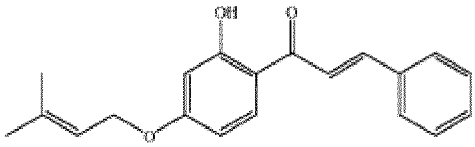, 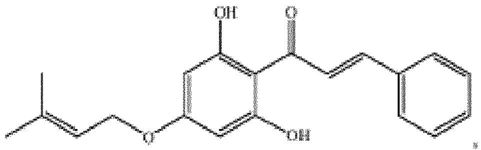,

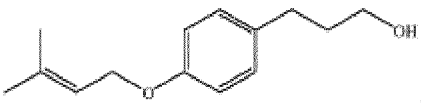, 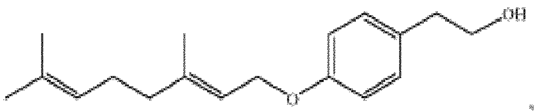,

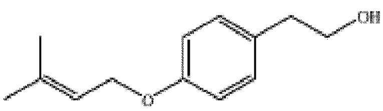, 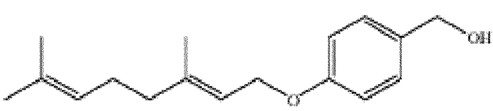,

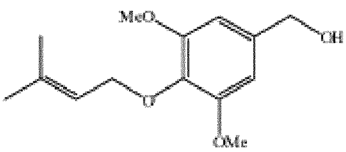, 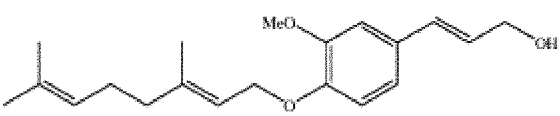,

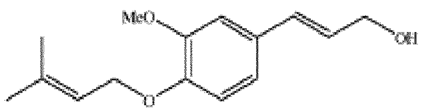,
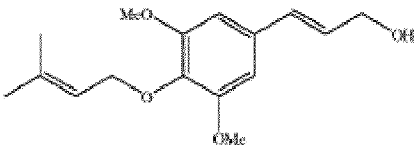,

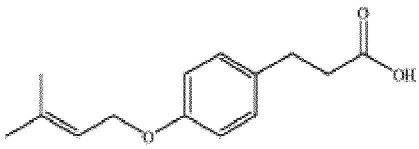,

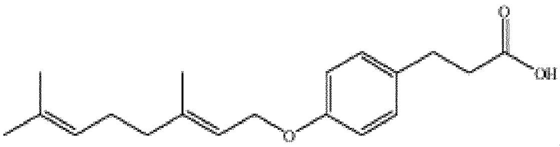,
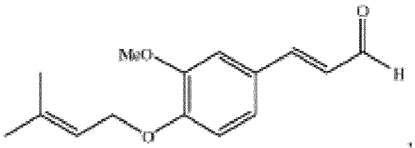,

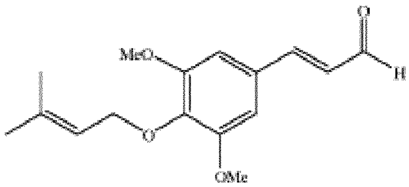,
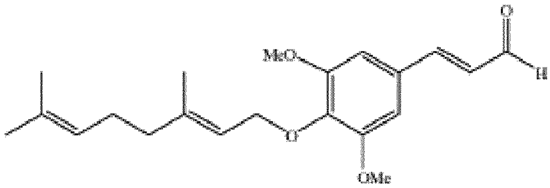,

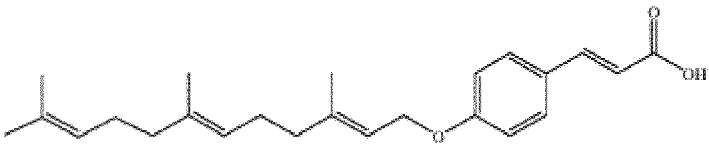,

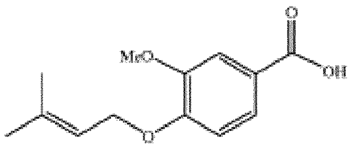,
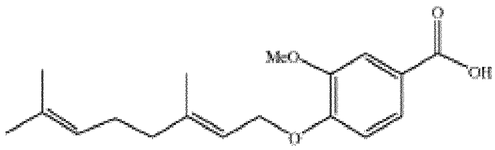

et des mélanges de ceux-ci.

**7.** Utilisation cosmétique d'un agent de dépigmentation de formule générale (I) selon l'une quelconque des revendications 1 à 6 pour induire un éclaircissement d'une peau ou d'une membrane muqueuse, améliorer l'uniformité et/ou la clarté d'une peau ou d'une membrane muqueuse, et/ou prévenir, réduire et/ou éliminer les points hyperpigmentés.

**8.** Composition cosmétique comprenant, dans un vecteur cosmétiquement acceptable, un agent de dépigmentation de formule générale (I) selon l'une quelconque des revendications 1 à 6, un extrait de plantes contenant ledit agent de dépigmentation de formule générale (I), et des mélanges de ceux-ci,
dans laquelle la composition cosmétique comprend en outre au moins un filtre UV, à condition que ledit au moins un filtre UV ne corresponde pas à la formule générale (II)

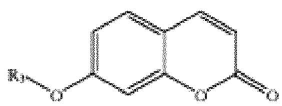

(II)

dans laquelle $R_3$ est un alcényle en $C_5$ à $C_{15}$ facultativement substitué par un hydroxy.

**9.** Composition cosmétique selon la revendication 8, dans laquelle ledit extrait de plantes est choisi dans le groupe constitué d'un extrait de plantes du genre *Ferula,* en particulier *Ferula szowitsiana, Ferula sinkiangensis, Ferula assa-foetida, Ferula tunetana, Ferula flabelliloba, Ferula fukanensis, Ferula persica* et *Ferula aitchisonii* ; d'un extrait de plantes du genre *Angelica,* en particulier *Angelica sylvestris, Angelica komarovii, Angelica pubescens, Angelica pachycarpa, Angelica decursiva, Angelica archangelica* et *Angelica ursina* ; d'un extrait de *Pimpinella anisum* ; d'un extrait de *Anethum graveolens* ; d'un extrait de *Ferulago campestris ;* d'un extrait de *Cicuta virosa* ; d'un extrait de *Seseli annuum* ; d'un extrait de graines de *Peucedanum zenkeri* ; d'un extrait de fruits de *Thapsia villosa* ; d'un extrait de *Scandix pecten-veneris* ; d'un extrait de *Scabiosa comosa* ; d'un extrait de *Heracleum sphondylium* ; d'un extrait de *Ammi majus* ; d'un extrait de *Xanthogalum sachokianum* ; d'un extrait de *Thamnosa montana* ; d'un extrait de *Ligusticum seguieri* ; et des mélanges de ceux-ci.

**10.** Composition cosmétique selon l'une quelconque de la revendication 8 ou 9, dans laquelle elle comprend de 0,0001 % à 50 %, en particulier de 0,1 % à 20 %, plus particulièrement de 1 % à 10 %, en poids d'un agent de dépigmentation de formule générale (I) sur la base du poids de la composition.

**11.** Composition cosmétique selon l'une quelconque des revendications 8 à 10, dans laquelle elle comprend en outre un second agent de dépigmentation, ou des mélanges de ceux-ci.

**12.** Composition cosmétique selon la revendication 11, dans laquelle le second agent de dépigmentation est choisi dans le groupe constitué des inhibiteurs d'enzymes de la mélanogénèse, des agents de chélation du cuivre, des antioxydants, des inhibiteurs du transfert des mélanosomes des mélanocytes aux kératinocytes, des exfoliants, des inhibiteurs de la production d'alpha-MSH, des inhibiteurs de la production d'endothéline, des inhibiteurs de la production d'hormone adrénocorticotrope (ATCH), et des mélanges de ceux-ci.

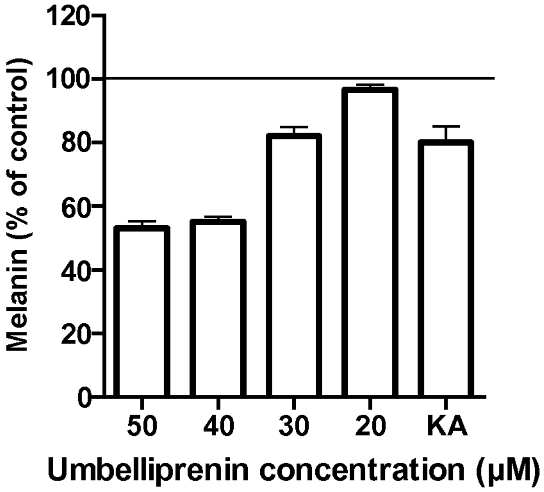


FIGURE 1

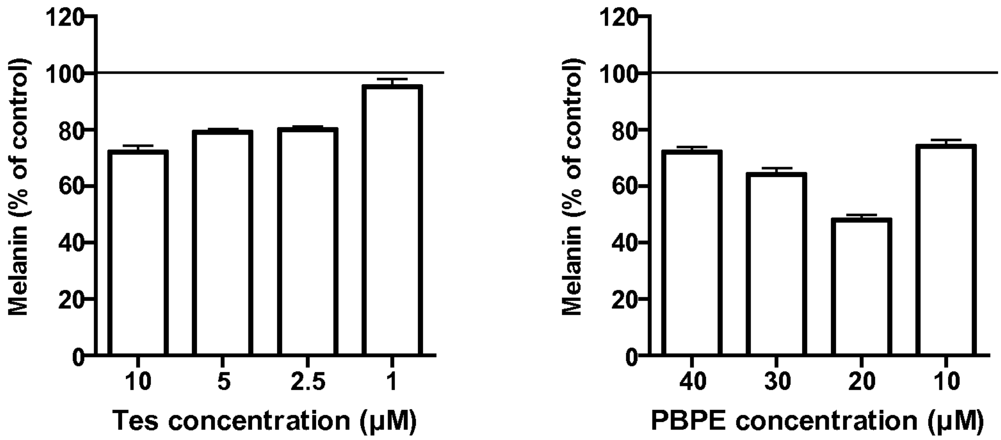


FIGURE 2

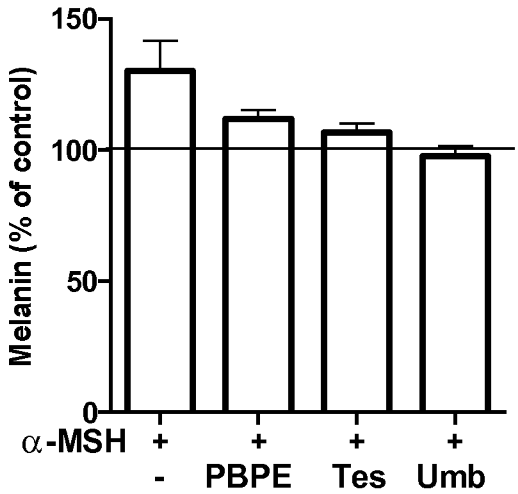


FIGURE 3

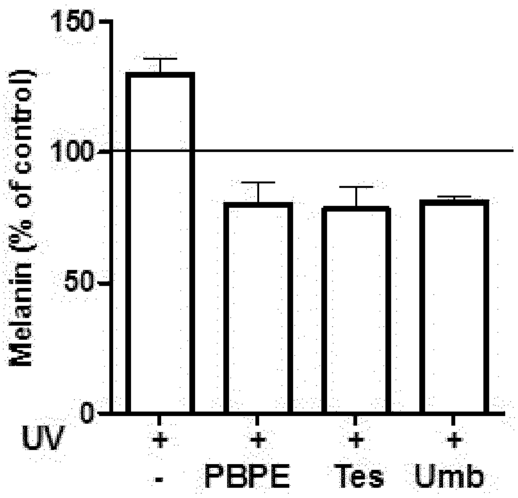


FIGURE 4

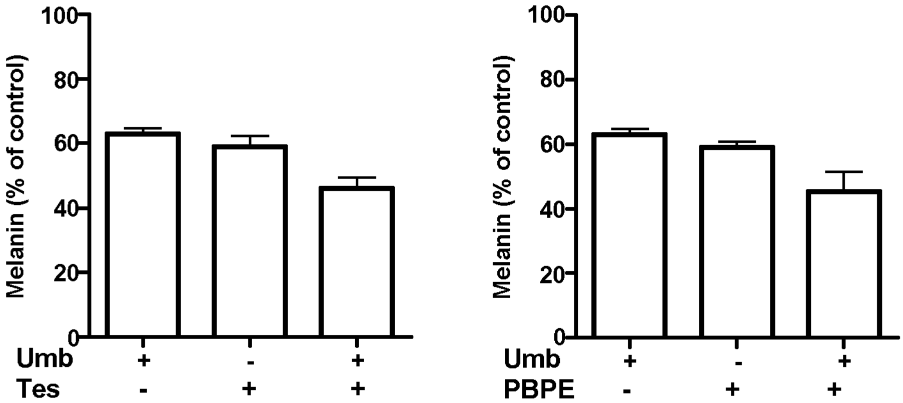


FIGURE 5

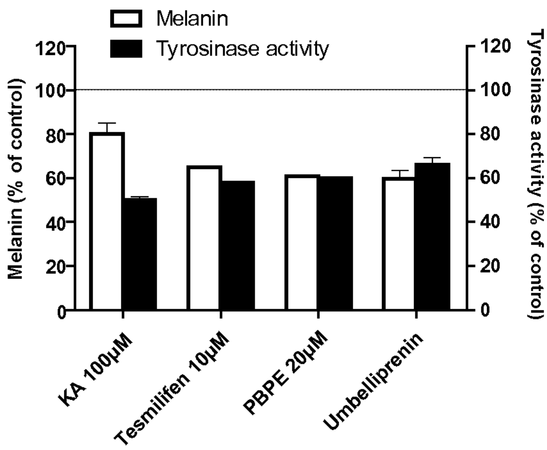


FIGURE 6

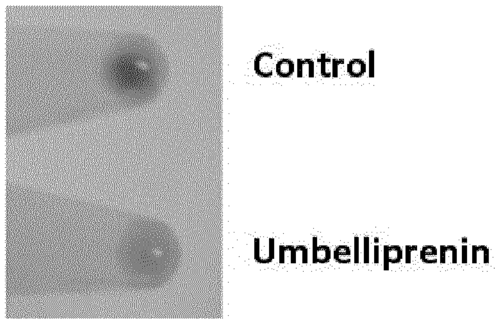


FIGURE 7

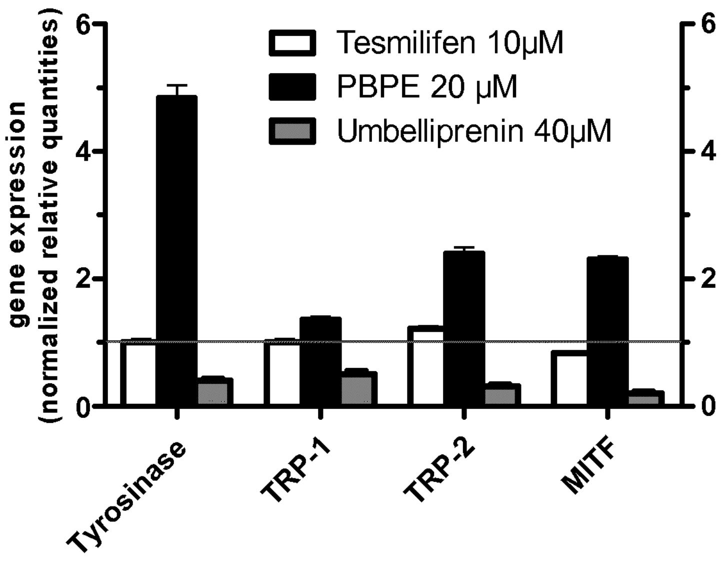


FIGURE 8

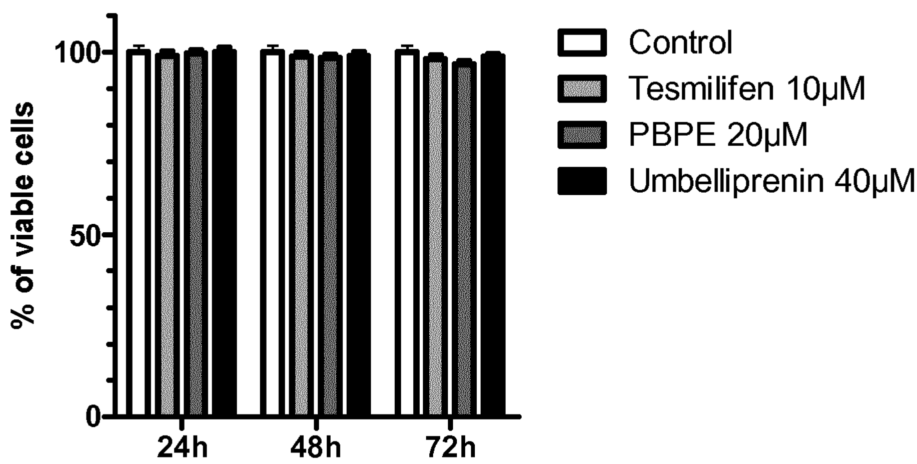


FIGURE 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012094638 A
- US 3712947 A


### Non-patent literature cited in the description


- *Nature Reviews, Molecular Cell Biology,* October 2001, vol. 2, 738-748
- CTFA Cosmetic Ingredient Handbook. 1992
- **POIROT M. et al.** *Bioorganic & Medicinal Chemistry,* 2000, vol. 8, 2007-2016
- **ANDO H.** *J. Lipid Res.,* 1999, vol. 40, 1312